# EUROPEAN PATENT APPLICATION

(11) **EP 4 233 868 A1**
(43) Date of publication of application: **30.08.2023**
(21) Application number: 21882109.8
(22) Date of filing: 21.10.2021
(51) Int. Cl.: A61K 31/519, A61K 39/395, A61P 35/00, A61P 37/00

(54) **USE OF FOLIC ACID AND FOLIC ACID-MODIFIED B CELL LYMPHOMA INDUCING B CELL IMMUNE TOLERANCE AND TARGETING MIGM POSITIVE EXPRESSION**

(30) Priority: 21.10.2020 CN 202011132019
(71) Applicant: Fudan University, Shanghai 200433 (CN)
(72) Inventor: ZHAN, Changyou, Shanghai 200032 (CN); WANG, Huan, Shanghai 200032 (CN); JIANG, Zhuxuan, Shanghai 200032 (CN)
(74) Representative: Bianchetti & Minoja with Trevisan & Cuonzo IPS SRL
(86) International application number: PCT/CN2021/125346
(87) International publication number: WO 2022/083694

(57) **Abstract**

Generally, the present invention relates to the use of folic acid and folate modification in inducing B-cell immune tolerance and targeting mIgM-positively-expressed B-cell lymphoma. The present invention also relates to a method, a pharmaceutical composition and a combination for inducing B-cell immune tolerance and for targeting mIgM-positively-expressed B-cell lymphoma.

## Description

### Technical Field

Generally, the present invention relates to the use of folic acid and folate modification in inducing B-cell immune tolerance and targeting mIgM-positively-expressed B-cell lymphoma, especially in reducing the production of the antibodies against an immunogenic substance, for the prevention or treatment of a disease or disorder that may be mediated by B-cell immune tolerance (e.g., hypersensitivity, autoimmune disease or transplant rejection), and/or for the targeted diagnosis, treatment or prevention of mIgM-positively-expressed B-cell lymphoma. The present invention also relates to a method, a pharmaceutical composition and a combination for inducing B-cell immune tolerance and for the targeted treatment, diagnosis or prevention of mIgM-positively-expressed B-cell lymphoma.

### Background

Biomacromolecular drugs, e.g., polypeptides and proteins, have significant effects in the treatment of immune-related diseases. However, such drugs have potential immunogenicity, and are prone to produce Anti-Drug Antibodies (ADAs), especially when used for a long time. As a result, the drugs are neutralized in the body, leading decreased concentration of drugs, inactivity of drugs, recurrence of diseases, and even infusion hypersensitivity. For example, in a phase I single-dose study of adalimumab, the incidence of ADAs in healthy subjects was as high as 70-100%. Inducing immune tolerance is one of the effective strategies to reduce ADAs and resistance to the biomacromolecular drugs. Immune tolerance is a state of specific immune hyporesponse or non-response after the body's immune system is exposed to certain antigen stimulation.

Moreover, inducing immune tolerance is also one of the most effective strategies for the prevention and treatment of a disease or disorder that may be mediated by B-cell immune tolerance, e.g., hypersensitivity, autoimmune disease or transplant rejection.

Nowdays, immunomodulators are mainly used to regulate the immune function of the body. However, it shall be administered for a long time and is accompanied by side effects, *e.g.,* easy activation of pathogens, tumor development, and increased infection probability.

The role of B-cell in the induction of immune tolerance has attracted more and more attention. B-cells are derived from pluripotent stem cells of the bone marrow. Mature B-cells migrate out through the peripheral blood, enter the spleen and lymph nodes, and are mainly distributed in the splenic nodules, splenic cord and lymph nodules, lymphatic cord and submucosal lymph nodules of the digestive tract. After being stimulated by antigen, mature B-cells differentiate and proliferate into plasma cells, synthesize antibodies, and exert humoral immune function. B-cells are the most numerous antigen-presenting cells in the body, and their characteristic surface markers are membrane surface immunoglobulin, which can be used as specific antigen receptors to recognize various antigenic epitopes. Membrane surface immunoglobulin M (mIgM) constitutes the antigen receptor of B-cell. Acting on mIgM through specific ligands can cause B-cell anergy and induce B-cell immune tolerance.

Therefore, there is a need in the art for new medical options that can specifically bind to mIgM receptors expressed on the surface of B-cell and induce B-cell immune tolerance, for reducing the production of the antibodies against an immunogenic substance and/or for the prevention or treatment of a disease or disorder that may be mediated by B-cell immune tolerance (e.g., hypersensitivity, autoimmune disease or transplant rejection) and/or for the targeted diagnosis, treatment or prevention of mIgM-positively-expressed B-cell lymphoma.

The present application satisfies the above needs through the technical solutions described below and in the appended claims.

### Description of Invention

### Brief Description of the Invention

Folic acid (FA), also known as pteroylglutamic acid, is composed of pterin, p-aminobenzoic acid and L-glutamic acid. Folic acid plays an important role in the body's metabolism. It participates in the synthesis of purine and ribonucleic acid, helps regulate the development of embryonic nerve cells, and prevents newborn babies from congenital neural tube defects. Folic acid has become a new health vitamin product in the international market after vitamins C and E. Due to such advantages as safety, stability and cheapness, it has a very broad market prospect.

The present inventors unexpectedly found through extensive researches that: folic acid specifically binds to mIgM expressed on the surface of B-cell, and then can target mIgM-positively-expressed B-cell, resulting in B-cell anergy and B-cell immune tolerance through inhibiting the maturation of B-cell and their differentiation to germinal center B-cell. Therefore, the administration of folic acid or a salt or ester or conjugate thereof can induce B-cell immune tolerance in the body, which can lead to specific immune hyporesponse or no response to the stimulation of immunogenic substances (*e.g.,* biomacromolecular drugs, immunogenic drug delivery systems, or pathogenic antigenic substances causing abnormal immune responses in the body, etc.), for reducing the production of the antibodies against an immunogenic substance and/or for treating or preventing a disease or disorder that may be mediated by B-cell immune tolerance. Moreover, the inventors also unexpectedly found that: the B-cell immune tolerance induced by folic acid or a salt or ester or conjugate thereof is reversible, and the immune function of the body recovered when the administration of folic acid or a salt or ester or conjugate thereof is discontinued, which is especially advantageous for the individuals who wish to be immune-tolerant only temporarily for medical purposes.

The inventors also unexpectedly found that: modifying the immunogenic substance with folate can significantly reduce the production of the antibodies against an immunogenic substance. For example, modifying the biomacromolecular active agents with folate can significantly reduce the autoimmunogenicity of biomacromolecular active agents, inhibiting Anti-Drug Antibodies (ADAs) and the resistance to the agents. Modifying a pathogenic antigenic substance causing abnormal immune responses in the body with folate can significantly reduce the production of antigen-specific antibodies, for preventing or treating a disease or disorder that may be mediated by B-cell immune tolerance, e.g., hypersensitivity, autoimmune disease and/or transplant rejection, etc.

Moreover, the inventors have also unexpectedly found that: modifying drugs (e.g, antitumor agents) or probes with folate can achieve the targeted diagnosis, treatment and/or prevention of mIgM-positively-expressed B-cell lymphoma through mIgM pathway.

Therefore, in a first aspect, the present invention provides the use of folic acid or a pharmaceutically acceptable salt or ester or conjugate thereof in the manufacture of a medicament for inducing B-cell immune tolerance, especially for reducing the production of the antibodies against an immunogenic substance, and/or for the treatment or prevention of a disease or disorder that may be mediated by B-cell immune tolerance.

In a second aspect, the present invention provides the use of folate-modified immunogenic substances in the manufacture of a medicament for inducing B-cell immune tolerance, especially for reducing the production of the antibodies against an immunogenic substance, and/or for the treatment or prevention of a disease or disorder that may be mediated by B-cell immune tolerance.

In the third aspect, the present invention provides the use of folate-modified antitumor agents or folate-modified probes in the manufacture of a medicament for the targeted diagnosis, treatment or prevention of mIgM-positively-expressed B-cell lymphoma.

In a fourth aspect, the present invention provides a method for inducing B-cell immune tolerance, especially for reducing the production of the antibodies against an immunogenic substance, and/or for the treatment or prevention of a disease or disorder that may be mediated by B-cell immune tolerance, comprising administrating an effective amount of folic acid or a pharmaceutically acceptable salt or ester or conjugate thereof or folate-modified immunogenic substance according to the invention to a subject.

In a fifth aspect, the present invention provides a method for the targeted diagnosis, treatment or prevention of mIgM-positively-expressed B-cell lymphoma, comprising administering an effective amount of folate-modified antitumor agent or folate-modified probe according to the invention to a subject.

In a sixth aspect, the present invention provides folic acid or a pharmaceutically acceptable salt or ester or conjugate thereof or folate-modified immunogenic substance according to the invention, for inducing B-cell immune tolerance, especially for reducing the production of the antibodies against an immunogenic substance, and/or for the treatment or prevention of a disease or disorder that may be mediated by B-cell immune tolerance.

In the seventh aspect, the present invention provides the folate-modified antitumor agent or folate-modified probe according to the invention, for the targeted diagnosis, treatment or prevention of mIgM-positively-expressed B-cell lymphoma.

In the eighth aspect, the present invention provides a pharmaceutical composition comprising an effective amount of folic acid or a pharmaceutically acceptable salt or ester or conjugate thereof and/or folate-modified immunogenic substance and/or folate-modified antitumor agent and/or folate-modified probe, and optionally one or more pharmaceutically acceptable excipients.

In a ninth aspect, the present invention provides a combination of folic acid or a pharmaceutically acceptable salt or ester or conjugate thereof with an immunogenic substance.

### Detailed Description of the Invention

The present invention provides following embodiments:
Embodiment 1. Use of folic acid or a pharmaceutically acceptable salt or ester or conjugate thereof in the manufacture of a medicament for inducing B-cell immune tolerance, especially for reducing the production of the antibodies against an immunogenic substance, and/or for the treatment or prevention of a disease or disorder that may be mediated by B-cell immune tolerance.
Embodiment 2. The use according to Embodiment 1, wherein the folic acid or a pharmaceutically acceptable salt or ester or conjugate thereof is selected from the group consisting of free folic acid, a pharmaceutically acceptable salt of folic acid, a pharmaceutically acceptable ester of folic acid, and a pharmaceutically acceptable conjugate of folic acid (*e.g.,* folate-albumin conjugate, folate-polyethylene glycol conjugate, etc.), or any combination thereof.
Embodiment 3. Use according to Embodiment 1 or 2, wherein the B-cell is the one expressing mIgM, especially the one highly expressing mIgM, more especially spleen B-cell or lymph node B-cell.
Embodiment 4. Use according to anyone of Embodiments 1-3, wherein the immunogenic substance is a biomacromolecular active agent, and the medicament is administered in combination with the biomacromolecular active agent, *e.g.,* before and/or during the administration of the biomacromolecular active agent, to reduce the production of Anti-Drug Antibodies against the biomacromolecule active agent;
   preferably, the biomacromolecule active agent is selected from the group consisting of: polypeptide drugs, *e.g.,* p53 activating peptide, melittin, scorpion venom peptide, antibacterial peptide, or insulin; or protein drugs, *e.g.,* antibody drugs and especially monoclonal or polyclonal antibody drugs, interferons, growth factors, growth factor inhibitors, enzymes, or albumins, *e.g.,* human serum albumin or ovalbumin, including murine monoclonal antibodies, chimeric monoclonal antibodies, humanized monoclonal antibodies, or fully human monoclonal antibodies, *e.g.,* Tumor Necrosis Factor α (TNFα) mAbs (*e.g.,* adalimumab, etanercept, or infliximab), PD1/PD-L1 mAbs (*e.g.,* nivolumab, pembrolizumab, atezolizumab, sintilimab, toripalimab, or camrelizumab); HER2 mAbs (*e.g.,* trastuzumab, pertuzumab, or lapatinib); CD20 mAbs (*e.g.,* rituximab, ibritumomab tiuxetan, or tositumomab); Vascular Endothelial Growth Factor/Vascular Endothelial Growth Factor Receptor (VEGF/VEGFR) mAbs (*e.g.,* bevacizumab, ranibizumab, aflibercept, or ramucirumab); and Epidermal Growth Factor Receptor (EGFR) mAbs (*e.g.,* cetuximab, panitumumab, or necitumumab);
   more preferably, the biomacromolecule active agent is selected from the group consisting of adalimumab, infliximab, atezolizumab, sintilimab, toripalimab, trastuzumab, albumins and insulin.
Embodiment 5. Use according to anyone of Embodiments 1-3, wherein the immunogenic substance is an immunogenic drug delivery system, *e.g.,* a microcarrier drug delivery system, preferably selected from the group consisting of liposomes, microspheres, microcapsules, nanoparticles, nanocapsules, lipid nanodiscs or polymer micelles, and wherein the medicament is administered in combination with the drug delivery system, *e.g.,* before and/or during the administration of the drug delivery system, to reduce the production of antibodies against the immunogenic drug delivery system; preferaly, the drug delivery system is loaded with the biomacromolecule active agent as defined in Embodiment 4.
Embodiment 6. Use according to anyone of Embodiments 1-3, wherein the medicament is used for the treatment or prevention of a disease or disorder that may be mediated by B-cell immune tolerance, *e.g.,* hypersensitivity, autoimmune disease or transplant rejection; preferably, the hypersensitivity is selected from the group consisting of anaphylactic shock, respiratory hypersensitivity (e.g., allergic asthma or allergic rhinitis) and gastrointestinal tract hypersensitivity; the autoimmune disease is selected from the group consisting of systemic lupus erythematosus, rheumatoid arthritis, Hashimoto's thyroiditis, toxic diffuse goiter, ankylosing spondylitis, autoimmune encephalomyelitis, neuromyelitis optica spectrum disorders, anticardiolipin syndrome, hemophilia and psoriasis; and the transplant rejection is selected from the group consisting of organ transplant rejection, tissue transplant rejection (*e.g.,* bone marrow transplant rejection) and recurrent miscarriage.
Embodiment 7. Use of folate-modified immunogenic substance in the manufacture of a medicament for inducing B-cell immune tolerance, especially for reducing the production of the antibodies against an immunogenic substance, and/or for the treatment or prevention of a disease or disorder that may be mediated by B-cell immune tolerance.
Embodiment 8. Use according to Embodiment 7, wherein the B-cell is the one expressing mIgM, especially the one highly expressing mIgM, more especially spleen B-cell or lymph node B-cell.
Embodiment 9. Use according to Embodiment 7 or 8, wherein the immunogenic substance is a biomacromolecule active agent;
   preferably, the biomacromolecule active agent is selected from the group consisting of: polypeptide drugs, *e.g.,* p53 activating peptide, melittin, scorpion venom peptide, antibacterial peptide, or insulin; or protein drugs, *e.g.,* antibody drugs and especially monoclonal or polyclonal antibody drugs, interferons, growth factors, growth factor inhibitors, enzymes, or albumins, *e.g.,* human serum albumin or ovalbumin, including murine monoclonal antibodies, chimeric monoclonal antibodies, humanized monoclonal antibodies, or fully human monoclonal antibodies, *e.g.,* Tumor Necrosis Factor α (TNFα) mAbs (*e.g.,* adalimumab, etanercept, or infliximab), PD1/PD-L1 mAbs (*e.g.,* nivolumab, pembrolizumab, atezolizumab, sintilimab, toripalimab, or camrelizumab); HER2 mAbs (*e.g.,* trastuzumab, pertuzumab, or lapatinib); CD20 mAbs (*e.g.,* rituximab, ibritumomab tiuxetan, or tositumomab); Vascular Endothelial Growth Factor/Vascular Endothelial Growth Factor Receptor (VEGF/VEGFR) mAbs (*e.g.,* bevacizumab, ranibizumab, aflibercept, or ramucirumab); and Epidermal Growth Factor Receptor (EGFR) mAbs (*e.g.,* cetuximab, panitumumab, or necitumumab);
   more preferably, the biomacromolecule active agent is selected from the group consisting of adalimumab, infliximab, atezolizumab, sintilimab, toripalimab, trastuzumab, albumins and insulin.
Embodiment 10. Use according to Embodiment 7 or 8, wherein the immunogenic substance is a pathogenic antigenic substance causing an abnormal immune response in the body, preferably a pathogenic antigenic substance causing hypersensitivity, autoimmune disease or transplant rejection, *e.g.,* ovalbumin, proteolipid protein, Dby polypeptide, Uty, myelin basic protein, denatured IgG, thyroglobulin, thyrotropin receptor, histone, acetylcholine receptor, major histocompatibility antigen (including HLA-I and HLA-II), minor histocompatibility antigens, blood group antigens, tissue-specific antigens (*e.g.,* vascular endothelial cell antigens, skin SK antigens).
Embodiment 11. Use according to Embodiment 10, wherein the medicament is used for the treatment or prevention of hypersensitivity, autoimmune disease or transplant rejection; preferably, the hypersensitivity is selected from the group consisting of anaphylactic shock, respiratory hypersensitivity (e.g., allergic asthma or allergic rhinitis) and gastrointestinal tract hypersensitivity; the autoimmune disease is selected from the group consisting of systemic lupus erythematosus, rheumatoid arthritis, Hashimoto's thyroiditis, toxic diffuse goiter, ankylosing spondylitis, autoimmune encephalomyelitis, neuromyelitis optica spectrum disorders, anticardiolipin syndrome, hemophilia and psoriasis; and the transplant rejection is selected from the group consisting of organ transplant rejection, tissue transplant rejection (*e.g.,* bone marrow transplant rejection) and recurrent miscarriage.
Embodiment 12. Use of folate-modified antitumor agent or folate-modified probe in the manufacture of a medicament for the targeted diagnosis, treatment or prevention of mIgM-positively-expressed B-cell lymphoma.
Embodiment 13. Use according to Embodiment 12, wherein the antitumor agent in the folate-modified antitumor agent is an antitumor agent useful for treating or preventing mIgM-positively-expressed B-cell lymphoma, preferably anthracyclines, *e.g.,* adriamycin or epirubicin; taxanes, *e.g.,* paclitaxel, docetaxel or cabazitaxel; camptothecins, *e.g.,* camptothecin, hydroxycamptothecin, 9-nitrocamptothecin or irinotecan; vinblastine drugs, *e.g.,* vincristine or vinorelbine; proteasome inhibitors, *e.g.,* bortezomib or carfilzomib; lactone drugs, *e.g.,* parthenolide; cyclophosphamides, etoposide, gemcitabine, cytarabine, 5-fluorouracil, teniposide, mubritinib, epothilone, actinomycin D, mitoxantrone, mitomycin, bleomycin, cisplatin, oxaliplatin, p53 activating peptide, melittin, scorpion venom peptide, triptolide, bevacizumab or trastuzumab; and wherein the probe in the folate-modified probe is a contrast agent, preferably fluorescent substance, *e.g.,* fluorescein, carboxyfluorescein (FAM), fluorescein isothiocyanate (FITC), hexachlorofluorescein (HEX), coumarin 6, near-infrared dyes Cy5, Cy5.5, Cy7, ICG, IR820, DiR or DiD; or radioactive substance, *e.g.,* magnetic resonance contrast agent, *e.g.,* Gd-DTPA or radiocontrast agent, *e.g.,* ⁹⁹mTc-DTPA.
Embodiment 14. A method for inducing B-cell immune tolerance, especially for reducing the production of the antibodies against an immunogenic substance, and/or for the treatment or prevention of a disease or disorder that may be mediated by B-cell immune tolerance, comprising administrating an effective amount of folic acid or a pharmaceutically acceptable salt or ester or conjugate thereof as defined in anyone of Embodiments 1-6 and/or folate-modified immunogenic substance as defined in anyone of Embodiments 7-11 to a subj ect.
Embodiment 15. A method for the targeted diagnosis, treatment or prevention of mIgM-positively-expressed B-cell lymphoma, comprising administering an effective amount of folate-modified antitumor agent or folate-modified probe as defined in Embodiment 12 or 13 to a subject.
Embodiment 16. Folic acid or a pharmaceutically acceptable salt or ester or conjugate thereof as defined in anyone of Embodiments 1-6 and/or folate-modified immunogenic substance as defined in anyone of Embodiments 7-11, especially folate-albumin conjugate, folate-polyethylene glycol conjugate, folate-modified human serumn albumin, folate-modified insulin, folate-modified adalimumab, folate-modified infliximab, folate-modified atezolizumab or folate-modified trastuzumab, for inducing B-cell immune tolerance, especially for reducing the production of the antibodies against an immunogenic substance, and/or for the treatment or prevention of a disease or disorder that may be mediated by B-cell immune tolerance.
Embodiment 17. Folate-modified antitumor agent or folate-modified probe, especially folate-modified cabazitaxel or folate-modified triptolide as defined in Embodiment 12 or 13, for the targeted diagnosis, treatment or prevention of mIgM-positively-expressed B-cell lymphoma.
Embodiment 18. A pharmaceutical composition comprising an effective amount of folic acid or a pharmaceutically acceptable salt or ester or conjugate thereof as defined in anyone of Embodiments 1-6 and/or folate-modified immunogenic substance as defined in anyone of Embodiments 7-11 and/or folate-modified antitumor agent or folate-modified probe as defined in Embodiments 12 or 13, and optionally one or more pharmaceutically acceptable excipients.
Embodiment 19. A combination of folic acid or a pharmaceutically acceptable salt or ester or conjugate thereof with an immunogenic substance, wherein the immunogenic substance is preferably a biomacromolecule active agent or an immunogenic drug delivery system;
   wherein, more preferably, the biomacromolecule active agent is selected from the group consisting of: polypeptide drugs, *e.g.,* p53 activating peptide, melittin, scorpion venom peptide, antibacterial peptide, or insulin; or protein drugs, *e.g.,* antibody drugs and especially monoclonal or polyclonal antibody drugs, interferons, growth factors, growth factor inhibitors, enzymes, or albumins, *e.g.,* human serum albumin or ovalbumin, including murine monoclonal antibodies, chimeric monoclonal antibodies, humanized monoclonal antibodies, or fully human monoclonal antibodies, *e.g.,* Tumor Necrosis Factor α (TNFα) mAbs (*e.g.,* adalimumab, etanercept, or infliximab), PD1/PD-L1 mAbs (*e.g.,* nivolumab, pembrolizumab, atezolizumab, sintilimab, toripalimab, or camrelizumab); HER2 mAbs (*e.g.,* trastuzumab, pertuzumab, or lapatinib); CD20 mAbs (*e.g.,* rituximab, ibritumomab tiuxetan, or tositumomab); Vascular Endothelial Growth Factor/Vascular Endothelial Growth Factor Receptor (VEGF/VEGFR) mAbs (*e.g.,* bevacizumab, ranibizumab, aflibercept or ramucirumab); and Epidermal Growth Factor Receptor (EGFR) mAbs (*e.g.,* cetuximab, panitumumab or necitumumab); still more preferably, the biomacromolecule active agent is selected from the group consisting of adalimumab, infliximab, atezolizumab, sintilimab, toripalimab, trastuzumab, albumins and insulin;
      or
   wherein, more preferably, the immunogenic drug delivery system is microcarrier drug delivery system, e.g., liposome, microsphere, microcapsule, nanoparticle, nanocapsule, lipid nanodisc or polymer micelle.

It will be appreciated that the features specified in each embodiment may be combined with any other specified features to provide other embodiments, and such combined embodiments are also within the scope of the application.

The terms and symbols used in the application have the meanings commonly understood by those skilled in the art, unless otherwise specified. Particularly, the following terms in the application have the meanings indicated below, unless otherwise stated.

As used herein, the expressions "a", "an", "the", "said" or the entities per se include both singular and plural forms, unless otherwise indicated.

As used herein, the terms "contain", "comprise", "include" and similar terms are to be understood to include unspecified materials in addition to those specified so long as they are compatible.

As used herein, the terms "and/or", when connecting two or more optional items, should be understood as referring to any one or more of the optional items.

As used herein, the term "about" is understood to mean an adjustment of a given value by 20%, *e.g.,* 10%, e.g., 5%, above or below that value.

As used herein, "folic acid or a salt or ester or conjugate" includes free folic acid, salt of folic acid, ester of folic acid, or folate conjugate (e.g., folate-albumin conjugate or folate-polyethylene glycol conjugates, etc.), or any combination thereof. Accordingly, as used herein, the term "folic acid or a pharmaceutically acceptable salt or ester or conjugate thereof' includes free folic acid or a pharmaceutically acceptable folate salt, folate ester or folate conjugate (e.g., folate-albumin conjugate or folate-polyethylene glycol conjugates, etc.), or any combination thereof.

As used herein, the term "folate modification" or "folate-modified" means that folate is linked with other substance, *e.g.,* immunogenic substances (*e.g.,* biomacromolecules or pathogenic antigen substances), antitumor agents, probes, polymer materials, etc., via chemical bonding to form complexes.

As used herein, the term "agent according to the invention" or "agents according to the invention" refers to one or more of folic acid or a pharmaceutically acceptable salt or ester or conjugate, folate-modified immunogenic substances (e.g., folate-modified biomacromolecule active agents, folate-modified pathogenic antigen substances), folate-modified antitumor agents or folate-modified probes as defined herein. Furthermore, the agent according to the invention may also refer to a combination of folic acid or a pharmaceutically acceptable salt or ester or conjugate thereof with an immunogenic substance.

As used herein, the term "immunogenic substance" refers to a substance that can activate and induce an immune response in the body, including but not limited to biomacromolecules, *e.g.,* biomacromolecule active agents, immunogenic drug delivery systems, or pathogenic antigenic material causing abnormal immune reaction in the body.

As used herein, the term "drug delivery system" refers to a system that regulates the distribution of drugs in the body in terms of space, time and/or dosage.

As used herein, the term "immunogenic drug delivery system" refers to a drug delivery system capable of activating and inducing an immune response in the body, *e.g.,* a microcarrier drug delivery system.

As used herein, the term "microcarrier drug delivery system" includes a system at micron, submicron or nanometer levels, *e.g.,* liposomes, microspheres, microcapsules, microemulsions, nanoparticles, nanocapsules, nanodiscs, polymeric micelles etc.

As used herein, the term "biomacromolecule" refers to a high molecular weight organic substance as an active ingredient in an organism, for example, having a molecular weight of thousands of daltons or higher, including but not limited to polypeptides or proteins, *e.g.,* antibodies.

As used herein, the terms "biomacromolecule drug" and "biomacromolecule active agent" are used interchangeably and refer to natural or synthetic biomacromolecules with therapeutic or prophylactic activity, e.g., including but not limited to polypeptide drugs or protein drugs, *e.g.,* antibodies drugs etc.

As used herein, the terms "pathogenic antigenic substance causing an abnormal immune response in the body" and "pathogenic antigenic substance" are used interchangeably and refer to an immunogenic substance that causes an excessively high immune response in the body, thereby causing tissue damage and/or manifesting disease state.

As used herein, the term "antitumor agent" refers to a substance useful in preventing or treating tumor diseases or cancers, *e.g.,* chemotherapeutic drug, hormone drug or toxin.

As used herein, the term "probe" refers to a functional chemical substance capable of detecting or displaying a biomedical problem, *e.g.,* diagnosing or treating a disease, *e.g.,* a contrast agent etc.

As used herein, the term "contrast agent" refers to a chemical substance administered to a tissue or organ of an individual in order to enhance the observation of an image.

As used herein, the term "pharmaceutically acceptable" means chemically / toxicologically compatible with other ingredients of the formulation or composition (including excipients and/or active ingredients) and/or compatible with the body.

As used herein, the term "pharmaceutically acceptable salt" refers to a salt formed by the parent compound with a pharmaceutically acceptable acid or base. Pharmaceutically acceptable salts are well known in the art, including but not limited to inorganic acid salts, *e.g.,* hydrochloride, sulfate, phosphate, etc.; organic acid salts, *e.g.,* acetate, propionate, methanesulfonate, ethanesulfonate, malate, citrate, tartrate, etc.; alkali metal or alkaline earth metal salts, *e.g.,* sodium, potassium, calcium salt, etc.

As used herein, the term "pharmaceutically acceptable ester" refers to a pharmaceutically acceptable ester derivative of a parent carboxylic acid, *e.g.,* a lower alkyl ester (e.g., methyl ester, ethyl ester, n-propyl ester or isopropyl ester, etc.), cycloalkyl esters, lower alkenyl esters, benzyl esters, *e.g.,* ω-(amino, mono- or di-lower alkylamino, carboxyl, lower alkoxy carbonyl)-lower alkyl esters, α-(lower alkanoyloxy, lower alkoxycarbonyl or di-lower alkyl aminocarbonyl)-lower alkyl esters, *e.g.,* pivaloyloxymethyl ester, and those conventionally used in the art.

As used herein, the term "conjugate" or "folate conjugate" refers to a complex formed by linking folic acid with other substance via chemical bond to improve the in vivo kinetic behavior and action time of folic acid, including but not limited to folate-albumin conjugate, folate-polyethylene glycol conjugate, etc.

As used herein, the term "pharmaceutically acceptable excipients" includes any and all pharmaceutically acceptable carriers or diluents, solvents, dispersion media, coatings, surfactants, antioxidants, preservatives, isotonic agents, absorption delaying agents, salts, preservatives, stabilizers, binders, excipients, disintegrants, lubricants, sweeteners, flavoring agents, dyes, or any combination thereof, as known to those skilled in the art. Any excipients known in the art can be used in the pharmaceutical composition or formulation, as long as it is compatible with the active ingredient.

As used herein, the term "B-cell" especially refers to a B-cell expressing mIgM, especially a B-cell highly expressing mIgM, *e.g.,* a spleen B-cell or a lymph node B-cell.

As used herein, the term "B-cell immune tolerance" means that the B-cell shows hyporesponse or no response to antigen stimulation, reduced immune response or no immune response.

As used herein, the term "antibodies against an immunogenic substance" refers to the specific antibodies induced by an immunogenic substance in the body.

As used herein, the term "Anti-Drug Antibodies" refers to Anti-Drug Antibodies (ADAs) induced by biomacromolecules with potential immunogenicity in the body. The production of ADAs can lead to decreased drug concentration, shortened half-life, reduced drug efficacy or even ineffectiveness, or hypersensitivity, and even life-threatening.

As used herein, the term "resistance" refers to a decrease in the effectiveness of a drug to treat a disease or ameliorate symptoms.

As used herein, the term "a disease or disorder that may be mediated by B-cell immune tolerance" refers to a disease or disorder that may benefit from, or be treated or prevented by, B-cell immune tolerance.

As used herein, the term "hypersensitivity" refers to adaptive immune response wherein the body is stimulated by certain antigens and produces such abnormality as physiological dysfunction or tissue cell damage.

As used herein, the term "autoimmune disease" refers to a disease state wherein the body produces abnormal immune response to self-antigens, resulting in self-cell destruction, tissue damage or abnormal function.

As used herein, the term "transplant rejection" means that, after a recipient has undergone a tissue or organ transplant, particularly an allogeneic or xenogeneic tissue or organ transplant, the foreign tissue or organ transplant is recognized as an foreign object by the recipient's immune system, which initiates an immunological response to attack, destruct and remove the transplant. The term "transplant rejection" also includes recurrent miscarriage.

As used herein, the terms "individual", "subject" and "patient" are used interchangeably and include any human or non-human animal. The term "non-human animal" includes all vertebrates, *e.g.,* mammals and non-mammals, *e.g.,* non-human primates, sheep, dogs, cats, horses, cows, chickens, amphibians, reptiles, etc. Preferably, the individual is a primate, especially a human.

As used herein, the term "effective amount" refers to the amount of the agent according to the invention which, when administered to cells or tissues of an individual or non-cellular biological materials or media, elicits a biological or medical response, *e.g.,* amelioration of symptoms, remission of disease, delay of a disease process, or prevention of a disease, etc. Those skilled in the art understand that the specific effective amount may vary depending on such factors as the intended biological endpoint, the drug to be delivered, and the target tissue, and that an "effective amount" may be administered in a single dose or in multiple doses.

As used herein, the terms "treat", "treatment" and the like refer to ameliorating, alleviating, reducing or arresting a disease or disorder or at least one clinical symptom thereof. In another embodiment, the terms "treat", "treatment" and the like refer to alleviating or ameliorating at least one body parameter, including those perceivable and/or non-perceivable by a patient. In another embodiment, the terms "treat", "treatment" and the like refer to physically modulating a disease or disorder (e.g., stabilizing perceivable symptoms) or physiologically modulating a disease or disorder (e.g., stabilizing a body parameter), or both. In another embodiment, the terms "treat", "treatment" and the like refer to arresting or delaying the onset or development or progression of a disease or disorder.

As used herein, the terms "prevent", "prevention" and the like refer to preventing the onset, development, or relapse of one or more symptoms of a disease or disorder in a subject by administering therapy (e.g., therapeutical agent) or therapy combination (e.g., a combination of therapeutical agents).

As used herein, the term "diagnosis" refers to making a judgment on the disease state of an individual after collecting information through such methods as examination or investigation.

As used herein, the term "combination" refers to the simultaneous, separate or sequential administration of two or more active agents via the same or different routes, which active agents may be included in the same pharmaceutical composition or in separate pharmaceutical compositions (e.g., a kit).

As used herein, the term "combination product" includes both the case where two or more active agents are in the same pharmaceutical composition, and the case where two or more active agents are in separate pharmaceutical compositions (e.g., a kit).

### Folic acid or a pharmaceutically acceptable salt or ester or conjugate thereof

Folic acid (FA), also known as pteroylglutamic acid, is composed of pterin, p-aminobenzoic acid and L-glutamic acid. The folic acid of the present invention may be of natural or synthetic origin.

A pharmaceutically acceptable salt or ester or conjugate of folic acid are commercially available, or can be prepared according to conventional methods in the art. For example, folate-albumin conjugates and folate-polyethylene glycol conjugates are commercially available from Ruixi Biological Technology Co., Ltd.

Administration of the folic acid or a pharmaceutically acceptable salt or ester or conjugate thereof according to the invention can cause B-cell immune tolerance in the body. Accordingly, folic acid or a pharmaceutically acceptable salt or ester or conjugate thereof according to the invention may be administered alone or in combination with an immunogenic substance, preferably before and/or during the administration of said immunogenic substance, to induce B-cell immune tolerance for reducing the production of the antibodies against an immunogenic substance and/or for treating or preventing a disease or condition that may be mediated by B-cell immune tolerance.

In one embodiment, the folic acid or a pharmaceutically acceptable salt or ester or conjugate thereof according to the invention are administered in a subject who is going to be administered, is being administered, or has been administered immunogenic substances (e.g., biomacromolecule active agents or immunogenic drug delivery systems) to reduce the production of the antibodies against an immunogenic substance. Preferably, folic acid or a pharmaceutically acceptable salt or ester or conjugate thereof is administered before and/or during the administration of the immunogenic substance. More preferably, the administration of the folic acid or a pharmaceutically acceptable salt or ester or conjugate thereof is administered (e.g., 15, 10, 9, 8, 7, 6, 5, 4, 3, 2, 1 day(s)) before the administration of the immunogenic substance and continues for years, months, weeks or days. Particularly, the administration of folic acid or a pharmaceutically acceptable salt or ester or conjugate thereof continues until the end of the administration of the immunogenic substance, *e.g.,* 3 months, 2 months, 1 month, 3 weeks, 2 weeks or 1 week thereafter, which depends on the properties, e.g., metabolism or half-life, of the immunogenic substance, and can be routinely determined by the participating physician.

In another embodiment, the folic acid or a pharmaceutically acceptable salt or ester or conjugate thereof according to the invention is administrated to a subject sufferring from a disease or disorder that may be mediated by B-cell immune tolerance (*e.g.,* hypersensitivity, autoimmune disease or transplant rejection) or at a risk thereof, to prevent the occurrence of said disease or disorder or to treat said disease or disorder, e.g., to alleviate one or more esymptoms thereof when said disease or disorder occurs.

### Immunogenic Substances

Immunogenic substances applicable in the present invention include, but are not limited to, biomacromolecules, *e.g.,* biomacromolecule active agents, immunogenic drug delivery systems, and pathogenic antigenic substances causing an abnormal immune response in the body.

In one embodiment, the immunogenic substance applicable in the present invention is a biomacromolecular active agent, including but not limited to polypeptide drugs, *e.g.,* p53 activating peptide, melittin, scorpion venom peptide, antibacterial peptide, or insulin; or protein drugs, *e.g.,* antibody drugs and especially monoclonal or polyclonal antibody drugs, interferons, growth factors, growth factor inhibitors, enzymes, or albumins, *e.g.,* human serum albumin or ovalbumin, including murine monoclonal antibodies, chimeric monoclonal antibodies, humanized monoclonal antibodies, or fully human monoclonal antibodies, *e.g.,* Tumor Necrosis Factor α (TNFα) mAbs (*e.g.,* adalimumab, etanercept or infliximab); PD1/PD-L1 mAbs (*e.g.,* nivolumab, pembrolizumab, atezolizumab, sintilimab, toripalimab or camrelizumab); HER2 mAbs (*e.g.,* trastuzumab, pertuzumab or lapatinib); CD20 mAbs (*e.g.,* rituximab, ibritumomab tiuxetan, or tositumomab); Vascular Endothelial Growth Factor/Vascular Endothelial Growth Factor Receptor (VEGF/VEGFR) mAbs (*e.g.,* bevacizumab, ranibizumab, aflibercept, or ramucirumab); or Epidermal Growth Factor Receptor (EGFR) mAbs (*e*.*g.,* cetuximab, panitumumab, or necitumumab).

More preferably, the biomacromolecule active agent applicable in the present invention is selected from the group consisting of adalimumab, infliximab, atezolizumab, sintilimab, toripalimab, trastuzumab, albumin and insulin.

In another embodiment, the immunogenic substance applicable in the present invention is an immunogenic drug delivery system, including but not limited to a microcarrier drug delivery system, preferably selected from liposomes, microspheres, microcapsules, nanoparticles, nanocapsules, lipid nanodiscs or polymer micelles. The drug delivery system may be loaded with any suitable drugs, *e.g.,* biomacromolecular active agents, antitumor agents, antibacterial agents, hormones and the like as described herein. When the drug delivery system is loaded with the biomacromolecule active agent as described herein, the folic acid or a pharmaceutically acceptable salt or ester or conjugate thereof according to the present invention can reduce both the antibodies against the drug delivery system and the ADAs against the biomacromolecule active agent. The preparation of the drug delivery systems is known in the art, for example, by film formation and hydration of macromolecule materials, *e.g.,* phospholipids or polymers.

In another embodiment, the immunogenic substance applicable in the present invention is a pathogenic antigenic substance causing an abnormal immune response in the body, preferably pathogenic antigenic substance causing a hypersensitivity, autoimmune disease or transplant rejection, *e.g.,* ovalbumin, proteolipid protein (PLP) polypeptide, Dby polypeptide, Uty, myelin basic protein, denatured IgG, thyroglobulin, thyrotropin receptor, histone, acetylcholine receptor, major histocompatibility antigens (including HLA-I and HLA-II), minor histocompatibility antigens, blood group antigens, tissue-specific antigens (*e.g.,* vascular endothelial cell antigens, skin SK antigens).

### Folate-modified Immunogenic Substances

The folate-modified immunogenic substance of the present invention may be prepared by forming a complex between folic acid and an immunogenic substance via a chemical bond or a linker.

In one embodiment, the folate-modified immunogenic agent may be a folate-modified biomacromolecular active agent. The administration of biomacromolecular active agents, especially for long term, is prone to produce Anti-Drug Antibodies and/or resistance. Preferably, the biomacromolecular drugs include but are not limited to those defined above. Particularly, the folate-modified biomacromolecule active agent of the present invention is folate-modified human serum albumin, folate-modified insulin, folate-modified adalimumab, folate-modified infliximab, folate-modified atezolizumab, folate-modified sintilimab, folate-modified toripalimab, or folate-modified trastuzumab. Folate modification can reduce the production of Anti-Drug Antibodies against biomacromolecular active agents.

In another embodiment, the folate-modified immunogenic agent according to the invention is a folate-modified pathogenic antigenic substance, wherein the substance causes an abnormal immune response in the body, *e.g.,* those defined above.

The method of modifying immunogenic substances with folate can be performed according to the methods known in the art or as exemplified in the examples below. For example, it may be prepared by condensing folate with biomacromolecules, *e.g.,* polypeptides or proteins, via direct chemical modification (e.g., amide condensation), bifunctional bridging groups, or solid-phase synthesis.

B-cell immune tolerance is induced by the folate modification of the present invention. Thus, the folate-modified immunogenic substances of the present invention are useful for reducing the production of the antibodies against an immunogenic substance and/or for the treatment or prevention of a disease or disorder that may be mediated by B-cell immune tolerance.

### Folate-modified Antitumor Agents

The folate-modified antitumor agent of the present invention may be prepared by forming a complex between folic acid and an antitumor agent via a chemical bond or a linker.

Antitumor agents applicable in the present invention are preferably those useful in the treatment or prevention of mIgM-positively-expressed B-cell lymphoma. Preferably, antitumor agents applicable in the present invention include but are not limited to anthracyclines, *e.g.,* adriamycin or epirubicin; taxanes, *e.g.,* paclitaxel, docetaxel or cabazitaxel; camptothecins, *e.g.,* camptothecin, hydroxycamptothecin, 9-nitrocamptothecin or irinotecan; vinblastine drugs, *e.g.,* vincristine or vinorelbine; proteasome inhibitors, *e.g.,* bortezomib or carfilzomib; lactone drugs, *e.g.,* parthenolide; cyclophosphamides, etoposide, gemcitabine, cytarabine, 5-fluorouracil, teniposide, mubritinib, epothilone, actinomycin D, mitoxantrone, mitomycin, bleomycin, cisplatin, oxaliplatin, p53 activating peptide, melittin, scorpion venom peptide, triptolide, bevacizumab, trastuzumab, etc. In certain embodiments, the folate-modified antitumor agent of the present invention is folate-modified cabazitaxel or folate-modified triptolide.

The preparation of the folate-modified antitumor agent of the present invention is known in the art or is exemplified in the examples. For example, the folate-modified antitumor agent of the present invention can be prepared as follows: (a) For drugs containing boronate groups, *e.g.,* bortezomib, folic acid is modified with dopamine and then reacted with the boronate group of the drug to form folate complexes comprising pH-sensitive borate; (b) For drugs containing ketone or aldehyde groups, *e.g.,* anthracyclines, *e.g.,* adriamycin or epirubicin, etc., folic acid is modified by introducing a sulfhydryl group and then reacted with maleimide hexylhydrazine derivative of the drug to form folate complexes comprising pH-sensitive hydrazone bond; (c) For drugs containing hydroxyl or amino groups, *e.g.,* paclitaxel, docetaxel, cabazitaxel, camptothecin, hydroxycamptothecin, 9-nitrocamptothecin, irinotecan, vincristine and vinorelbine, etc., folic acid is modified by introducing a sulfhydryl group and then reacted with 3-(2-pyridyldithiol)propionic acid derivative of the drug to form folate complexes comprising pH-sensitive disulfide bond; (d) For drugs containing active amino groups, *e.g.,* aminophosphates, aminoglycosides, adriamycin, etc., it can be directly formed by amide condensation; and (e) For drugs containing active carboxyl groups, *e.g.,* chlorambucil, it can be formed by introducing amino groups into folic acid followed by amide condensation.

The folate-modified antitumor agents of the present invention are useful in the targeted treatment or prevention of mIgM-positively-expressed B-cell lymphoma.

### Folate-modified Probes

The folate-modified probes of the invention may be prepared by forming a complex between folic acid and the probe via a chemical bond or a linker.

The probes applicable in the present invention may be contrast agentS, including but not limited to fluorescent substances, *e.g.,* fluorescein, carboxyfluorescein (FAM), fluorescein isothiocyanate (FITC), hexachlorofluorescein (HEX), coumarin 6, near-infrared dyes Cy5, Cy5.5, Cy7, ICG, IR820, DiR or DiD etc.; and radioactive substances, *e.g.,* magnetic resonance contrast agents, *e.g.,* Gd-DTPA or radiocontrast agents, *e.g.,* ⁹⁹mTc-DTPA, etc. Preferably, the probes applicable in the present invention are carboxyfluorescein (FAM), near-infrared dye Cy5, Cy5.5, ICG, IR820, DiR, DiD or Gd-DTPA, etc.

The preparation of the folate-modified probes is known in the art or is exemplified in the Examples below. For example, the folate-modified probes of the present invention may be prepared by amide condensation, functional derivatization, chelation, etc.

The folate-modified probes of the present invention are useful in the targeted diagnosis, treatment or prevention of mIgM-positively-expressed B-cell lymphoma.

### Combination of Folic Acid or a Pharmaceutically Acceptable Salt or Ester or Conjugate thereof with Immunogenic Substances

Co-administration of folic acid (free or in the form of a pharmaceutically acceptable salt or ester or conjugate) with an immunogenic substance reduces the production of the antibodies against an immunogenic substance.

In one embodiment, the immunogenic substance is preferably a biomacromolecular active agent, *e.g.,* those described above.

In another embodiment, the immunogenic substance is an immunogenic drug delivery system, *e.g.,* a microcarrier drug delivery system, preferably liposomes, microspheres, microcapsules, nanoparticles, nanocapsules, lipid nanodiscs or polymer micelles. The drug delivery system may be loaded with any suitable drugs, *e.g.,* biomacromolecular active agents, antitumor agents, antibacterial agents, hormones, etc. as described herein. Further, the immunogenic substance is a drug delivery system loaded with biomacromolecule active agents.

The administration of folic acid or a pharmaceutically acceptable salt or ester or conjugate thereof and the administration of the immunogenic substance may be performed simultaneously, sequentially or alternately. For example, folic acid or a pharmaceutically acceptable salt or ester or conjugate thereof is administered before and/or during the administration of the immunogenic substance. Preferably, folic acid or a pharmaceutically acceptable salt or ester or conjugate thereof is administered (e.g., 15, 10, 9, 8, 7, 6, 5, 4, 3, 2, 1 day(s)) before the administration of the immunogenic substance and continues for years, months, weeks or days. Particularly, the administration of folic acid or a pharmaceutically acceptable salt or ester or conjugate thereof continues until the end of the administration of the immunogenic substance, *e.g.,* 3 months, 2 months, 1 month, 3 weeks, 2 weeks or 1 week thereafter, which depends on the properties, *e.g.,* metabolism or half-life, of the immunogenic substance, and can be routinely determined by the participating physician. More preferably, the administration of folic acid or a pharmaceutically acceptable salt or ester or conjugate thereof and the administration of the immunogenic substance are performed simultaneously.

The routes of administration of folic acid or a pharmaceutically acceptable salt or ester or conjugate thereof may be the same with or different from that of the immunogenic substance. Preferably, folic acid or a pharmaceutically acceptable salt or ester or conjugate thereof may be administered by oral administration, inhalation, injection, etc., while the immunogenic substance may be administered in the routes of administration that is the same with or different from that of the immunogenic substance. Further, folic acid or a pharmaceutically acceptable salt or ester or conjugate thereof and the immunogenic substance may be included in the same pharmaceutical composition, or be included in separate pharmaceutical compositions. For example, folic acid or a pharmaceutically acceptable salt or ester or conjugate thereof and the immunogenic substance may be presented in a combination product, e.g., a kit.

### Pharmaceutical Composition and Administration

The agents according to the invention may be administered by any suitable route in the art, including but not limited to oral, parenteral such as subcutaneous, intramuscular, intravenous, intraperitoneal, inhalation, intranasal, rectal, transdermal, implantation, or topical routes. In certain embodiments, the agents according to the invention are administered by injection, e.g., continuous infusion, subcutaneous injection, intramuscular injection, or intravenous injection. In other embodiments, the agents according to the invention may be administered orally. In stilll other embodiments, the agents according to the invention may be administered by inhalation. Further, the agents according to the invention may be administered via implanted devices.

The agents according to the invention may be prepared into pharmaceutical preparations or pharmaceutical compositions by any appropriate procedures in the art. Pharmaceutical preparations or pharmaceutical compositions may be in solid forms (including but not limited to tablets, lozenges, coated tablets, capsules, pills, granules, cachets, lyophilizates, powders or suppositories, etc.), liquid forms (including but not limited to solutions, suspensions or emulsions, etc.) or semi-solid forms (including but not limited to creams, ointments, pastes, gels, etc.), for any suitable route in the art, *e.g.,* oral, parenteral such as subcutaneous, intramuscular, intravenous, intraperitoneal, inhalation or nasal, rectal, transdermal, implantation, topical administration, etc. Pharmaceutical preparations contain an effective amount of the agents according to the invention and optionally one or more pharmaceutically acceptable excipients, *e.g.,* diluents, isotonic agents, buffers, preservatives, stabilizers, wetting agents, emulsifiers, lubricants etc., as long as they are compatible. The pharmaceutical preparation or pharmaceutical composition may also be subjected to any suitable conventional pharmaceutical operations, *e.g.,* granulation, tablet compression, freeze-drying, sterilization, etc.

In certain embodiments, the pharmaceutical composition or formulation is in a form suitable for oral administration, *e.g.,* tablet, coated tablet, lozenge, capsule (soft or hard), powders, granules, cachet, emulsion, aqueous or oily suspensions, syrups or elixirs, etc.

In other embodiments, the pharmaceutical composition or formulation is in a form suitable for injectable administration, including but not limited to sterile aqueous solutions (where water soluble) or dispersions and sterile powders for extemporaneously preparing into sterile injectable solutions or dispersions before administration, preferably aqueous isotonic solutions or suspensions. Preferably, the pharmaceutical composition or formulation is for continuous infusion, subcutaneous injection, intramuscular injection or intravenous injection. The formulation components and methods suitable for injection are known in the art, and include, for example, physiological saline, stabilizers, buffers, isotonic agents, etc. In all cases, the composition should be sterile and should be flowable to the extent that easy syringability exists immediately before use. The pH of the formulation is usually about 3 to 11, more preferably about 5 to 9 or 6 to 8, most preferably about 7 to 8, e.g., about 7 to 7.5.

In other embodiments, the pharmaceutical composition or formulation is in a form suitable for inhalation or nasal administration. They may conveniently be delivered in aerosol or spray form from a pressurized container, pump, sprayer, atomizer or dispenser with or without suitable propellants, or in dry powder form (alone or as a mixture, *e.g.,* a dry mix with lactose) by a dry powder inhaler. Methods and components suitable for the preparation of pharmaceutical compositions or formulations for inhalation or nasal administration are known in the art. Particularly, in the case of aerosols, it preferably comprises, for example, a hydrofluoroalkane (HFA) propellant, *e.g.,* dichlorodifluoromethane, HFA134a or HFA227 or any mixture thereof, and may contain one or more co-solvents (*e.g.,* ethanol) and/or one or more surfactants (*e.g.,* oleic acid or sorbitan trioleate) and/or one or more fillers (*e.g.,* lactose) that are known in the art. When the pharmaceutical composition or formulation is a spray, it preferably contains an active agent dissolved or suspended in a vehicle comprising water, a solubilizer (*e.g.,* ethanol or propylene glycol) and a stabilizer (which may be a surfactant). When the pharmaceutical composition or formulation is a dry powder formulation, it preferably contains, for example, an active agent having a particle size of up to 10 microns, optionally with a diluent or carrier (*e.g.,* lactose) having the desired particle size distribution and the compounds (*e.g.,* magnesium stearate) that help prevent product performance degradation caused by moisture.

In other embodiments, the pharmaceutical composition or formulation is in a form suitable for transdermal or topical administration, including powders, sprays, ointments, pastes, creams, lotions, gels, solutions, patches, and inhalant. The agents according to the invention can be prepared into preparations by mixing with a pharmaceutically acceptable carrier and with any excipients that may be required, *e.g.,* penetration enhancers, preservatives or buffers, under sterile conditions.

In certain embodiments, the agents according to the invention may be administered intermittently or continuously, for example, via an implanted device or catheter, for one or several days or longer, *e.g.,* several weeks, months or years, *e.g.,* at least about 3 days, at least about 1 week, at least about 2 weeks, or at least 1 month.

The agents according to the invention are generally effective over a wide dosage range. For example, the daily dosage may be about 0.01-2000 mg, preferably about 1-1000 mg, more preferably about 2-100 mg, and most preferably about 5-80 mg. Dosages below the lower limit of the above dosage range may be employed in some cases, while the dosages above the upper limit of the above dosage range may be employed in other cases. Accordingly, the above dosage ranges are not intended to limit the scope of the invention in any way. It will be appreciated that the actual dosage will be determined by the physician taking into account the relevant circumstances, including the disease to be treated, the route of administration, the active agent(s) actually administered, the age, weight and response of a patient, and the severity of the patient's symptoms. Furthermore, the agents according to the invention may be administered as a single dose or as two or more doses (each dose may be the same or different).

The pharmaceutical formulation or composition of the invention may also contain other active agents, provided they are compatible. The other active agent may have the same or different pharmacological activity as the agents according to the invention.

### Pharmacological Efficacy and Use

The agents according to the invention are useful in diagnosis, treatment and/or prevention. Particularly, the agents according to the invention is able to specifically bind mIgM on the surface of B-cell and target B-cell expressing mIgM, especially B-cell highly expressing mIgM, leading to anergy of B-cell and inducing B-cell immune tolerance.

Accordingly, in one respect, the agents according to the invention are useful for inducing B-cell immune tolerance, especially for reducing the production of the antibodies against an immunogenic substance and/or for treating or preventing a disease or disorder that may be mediated by B-cell immune tolerance, including but not limited to hypersensitivity, autoimmune disease or transplant rejection. Preferably, the B-cell is the one expressing mIgM, particularly the one highly expressing mIgM, and more particularly a spleen B-cell or a lymph node B-cell.

In certain embodiments, the agents according to the invention, particularly folic acid or a pharmaceutically acceptable salt or ester or conjugate thereof or folate-modified biomacromolecular active agents, are useful for reducing Anti-Drug Antibodies against the biomacromolecular active agents (*e.g.,* polypeptides or proteins, *e.g.,* antibody drugs) and/or the resistance to the biomacromolecular active agents. Biomacromolecular drugs are potentially immunogenic, and are prone to produce Anti-Drug Antibodies (ADAs) especially when used for a long time. By administering a combination of folic acid or a pharmaceutically acceptable salt or ester or conjugate thereof with a biomacromolecule or by administering a folate-modified biomacromolecule, B-cell immune tolerance can be induced to make the body tolerant to the biomacromolecule, effectively reducing the production of Anti-Drug Antibodies and improving efficacy and safety. For example, long-term administration of adalimumab causes the body to produce antibodies against adalimumab. When adalimumab is administered again, the Anti-Drug Antibodies quickly neutralize adalimumab, resulting in a decrease in concentration and inactivity of the drug and inducing immune side effects. When folate-modified adalimumab is administered, the body is tolerant to adalimumab, which effectively reduces the production of Anti-Drug Antibodies and improves efficacy and safety.

In other embodiments, the agents according to the invention, particularly folic acid or a pharmaceutically acceptable salt or ester or conjugate thereof, are useful for reducing the production of antibodies against immunogenic drug delivery systems. For example, liposomes are commonly used drug delivery systems. However, repeated injections of liposomes can cause the body to produce antibodies against the liposomes, resulting in rapid clearance of liposomes from the body. By combining folic acid or a pharmaceutically acceptable salt or ester or conjugate thereof with liposomes, the production of the antibodies against liposomes can be effectively reduced, increasing the exposure of liposomes to the body.

In other embodiments, the agents according to the invention are useful in the treatment or prevention of hypersensitivity, e.g., by administering folic acid or a pharmaceutically acceptable salt or ester or conjugate thereof or by administering folate-modified pathogenic antigenic substances (*e.g.,* polypeptides or protein), wherein the pathogenic antigenic substances cause hypersensitivity. The hypersensitivity includes, but is not limited to, anaphylactic shock, respiratory tract hypersensitivity (e.g., allergic asthma or allergic rhinitis), gastrointestinal tract hypersensitivity, etc.

In other embodiments, the agents according to the invention are useful in the treatment or prevention of autoimmune disease, including but not limited to systemic lupus erythematosus, rheumatoid arthritis, Hashimoto's thyroiditis, toxic diffuse goiter, ankylosing spondylitis, autoimmune encephalomyelitis, neuromyelitis optica spectrum disorders, anticardiolipin syndrome, hemophilia, psoriasis, etc., for example by administering folic acid or a pharmaceutically acceptable salt or ester or conjugate thereof or by administering folate-modified pathogenic antigenic substances (*e.g.,* polypeptides or protein), wherein the pathogenic antigenic substances cause autoimmune disease. The pathogenic antigenic substances causing autoimmune disease include but are not limited to PLP polypeptide, myelin basic protein, denatured IgG, thyroglobulin, thyrotropin receptor, histone and acetylcholine receptor.

In other embodiments, the agents according to the invention are useful in the treatment or prevention of transplant rejection, for example by administering folic acid or a pharmaceutically acceptable salt or ester or conjugate thereof or by administering folate-modified pathogenic antigenic substances, wherein the pathogenic antigenic substances cause transplant rejection. For example, Dby (NAGFNSNRANSSRSS) and Uty (WMHHNMDLI)) are important antigens in mediating bone marrow transplantation rejection in female mice. Stimulating a recipient mouse with folate-modified Dby or Uty in advance can induce immune tolerance and then increase the success rate of transplantation of bone marrow from a donor mouse. The transplant rejection includes organ transplant rejection (e.g., but not limited to heart, liver, spleen, lung, or kidney transplant rejection), tissue transplant rejection (e.g., bone marrow transplant rejection) and recurrent miscarriage. The pathogenic antigenic substances causing transplant rejection include but are not limited to major histocompatibility antigens (including HLA-I, HLA-II), minor histocompatibility antigens, blood group antigens, tissue-specific antigens (e.g., vascular endothelial cell antigen, skin SK antigen), etc.

In another aspect, the agents according to the invention are useful in the targeted diagnosis, treatment or prevention of mIgM-positively-expressed B-cell lymphoma. By specifically recognizing the mIgM receptors expressed on the surface of B-cell lymphoma, the agents according to the invention are able to target mIgM-positively-expressed B-cell lymphoma, and achieve the targeted diagnosis, treatment or prevention of mIgM-positively-expressed B-cell lymphoma.

### Brief Description of Figures

**Fig. 1** shows the nuclear magnetic resonance spectrum of folate-OVA complex of Example 1.
**Fig. 2** shows a quantitative standard curve for the folic acid molecule.
**Fig. 3** shows the nuclear magnetic resonance spectrum of folate-PEG₂₀₀₀-Cy5 of Example 3.
**Fig. 4** shows the binding mode of folate with mouse mIgM in Example 4 (a: binding site of folate with mIgM; b: analysis on the binding force of folate to mIgM).
**Fig. 5** shows the results of flow cytometric analysis of the binding of folate to mouse mIgM in Example 4 (a: the effect of anti-IgM antibody to eliminate mIgM on the surface of B-cell; b: the results of the uptake of folate by B-cell under different anti-IgM antibody concentrations; c: Linear fitting curve of B-cell uptake of folate and mIgM expression level).
**Fig. 6** shows confocal micrographs of folate binded to mouse mIgM in Example 4.
**Fig. 7** shows the distribution of FA-PEG₂₀₀₀-Cy5 or PEG₂₀₀₀-Cy5 in the spleen 1 hours (a) and 4 hours (b) after injection via tail vein in Example 5. (^{∗} p<0.05, ^{∗∗}p<0.01, T test)
**Fig. 8** shows the flow cytometry graphs (a: 1h; c: 4h) and statistical analysis (b: 1h; d: 4h) of spleen lymphocytes 1 hours and 4 hours after injection of FA-PEG₂₀₀₀-Cy5 or PEG₂₀₀₀-Cy5 via tail vein in Example 5. (ns: no statistical difference, ^{∗∗∗}p<0.001, T test)
**Fig. 9** shows the immunofluorescence photographs of the distribution of FA-PEG₂₀₀₀-Cy5 or PEG₂₀₀₀-Cy5 in the spleens 1 hour after injection via tail vein in Example 5.
**Fig. 10** shows the immunofluorescence photographs of the binding of FA-PEG₂₀₀₀-Cy5 to IgM-positivly-expressed marginal B-cell in the spleen 1 hour after injection via tail vein in Example 5.
**Fig. 11** shows the B-cell proliferation (a, b) and differentiation (c, d) results obtained by flow cytometry after stimulating the mice with FA-OVA, OVA and physiological saline respectively for three times in Example 6. (*p<0.05, ***p<0.001, one-way ANOVA)
**Fig. 12** shows the expression of serum OVA-specific antibodies after stimulating the mice with FA-OVA, OVA and physiological saline respectively for three times in Example 6 (**a:** IgE; **b:** IgG; c: IgG1; **d:** IgG2a).
**Fig. 13** shows the expression of serum FVIII-specific antibodies after stimulating the mice with FVIII, folate-FVIII and physiological saline respectively for three times in Example 6.
**Fig. 14** shows the expression of OVA-specific IgG antibodies in mouse serum after oral administration of folic acid in Example 7 (**a:** day 14; **b:** day 21; **c:** day 28).
**Fig. 15** shows the expression of KLH-specific IgG antibodies in mouse serum after oral administration of folic acid in Example 7 (**a:** day 21; **b:** day 28; **c:** day 35).
**Fig. 16** shows the expression of serum-specific antibodies after stimulating the mice in Example 8 (**a:** adalimumab as a model mAb; b: infliximab as a model mAb; c: trastuzumab as a model mAb).
**Fig. 17** shows the expression of serum sLip-specific antibodies after stimulating the mice with sLip, sLip+FA (10µg), sLip+FA (50µg) and physiological saline in Example 9, respectively.
**Fig. 18** shows the body temperature change curves of the mice which was treated with FA-OVA, OVA and physiological saline respectively and then was challenged with OVA in Example 10.
**Fig. 19** shows the level of the serum OVA-specific antibodies in the mice which was treated with FA-OVA, OVA and physiological saline respectively and then was challenged with OVA in Example 10 (**a:** IgE; **b:** IgG₁, **c:** IgG₂ₐ).
**Fig. 20** shows the body temperature change curves of the sensitized mice which was treated with FA-OVA, OVA and physiological saline respectively and then was challenged with OVA in Example 11 (ns: no statistical difference, ^{∗∗}p<0.01, ^{∗∗∗}p<0.001, one-way ANOVA)
**Fig. 21** shows the levels of serum OVA-specific antibodies in the sensitized mice which was treated with FA-OVA, OVA and physiological saline respectively and then was challenged with OVA in Example 11 (**a:** IgE; **b:** IgG_{2a:} **c:** IgGi) (ns: no statistical difference, ***p<0.001, one-way ANOVA).

### Examples

The present invention is further illustrated below in combination with the specific examples. It should be understood that these examples are only illustrative and are not intended to limit the scope of the present invention. The experimental materials and reagents are commercially available or are parared according to methods known in the art, unless otherwise specified. Abbreviations used herein have the meanings commonly understood in the art, unless otherwise indicated.

Particularly, the abbreviations herein have the following meanings:

| **abbreviations** | **meanings** |
|---|---|
| BSA | bovine serum albumin |
| Chol | cholesterol |
| DAPI | fluorescent dye 4',6-diamidino-2-phenylindole |
| Dby | NAGFNSNRANSSRSS |
| DCC | dicyclohexyl carbodiimide |
| DMSO | dimethyl sulfoxide |
| DSPE | distearoylphosphatidylethanolamine |
| EDC | carbodiimide |
| EDTA | ethylenediamine tetraacetic acid |
| FA | folic acid |
| FVIII | coagulation factor VIII |
| h | hour(s) |
| HPLC | high-performance liquid chromatography |
| HSPC | hydrogenated soybean phospholipid |
| mPEG | polyethylene glycol monomethyl ether |
| NHS | N-hydroxysuccinimide |
| OVA | ovalbumin |
| PBS | phosphate buffer solution, pH=7.4 |
| PEG | polyethylene glycol |
| PLGA | poly(lactic-co-glycolic acid) |
| PLP | Proteolipid protein |
| POPC | 1-palmitoyl-2-oleoyl lecithin |
| TEA | triethanolamine |
| TLC | thin-layer chromatography |

### Example 1: Preparation of folate-protein complex

Folate-OVA was synthesized as an example of folate-modified protein complexes. Folic acid (4.4 mg) was dispersed in 3mL PBS (pH=7.4), and carbodiimide (EDC) (15 mg) was added. The mixture was stirred for 3h in dark to activate folic acid. Ovalbumin (OVA) (4.3 mg) was fully dissolved in 0.2mL PBS. Then, the activated folic acid solution was added dropwise into the OVA solution. The mixture was stirred for 3h in dark, and shaken overnight at 4°C. The next day, the mixture was dialyzed against 10 mM PBS for 72h, changing the dialysate every 12h. The product was obtained by lyophilization.

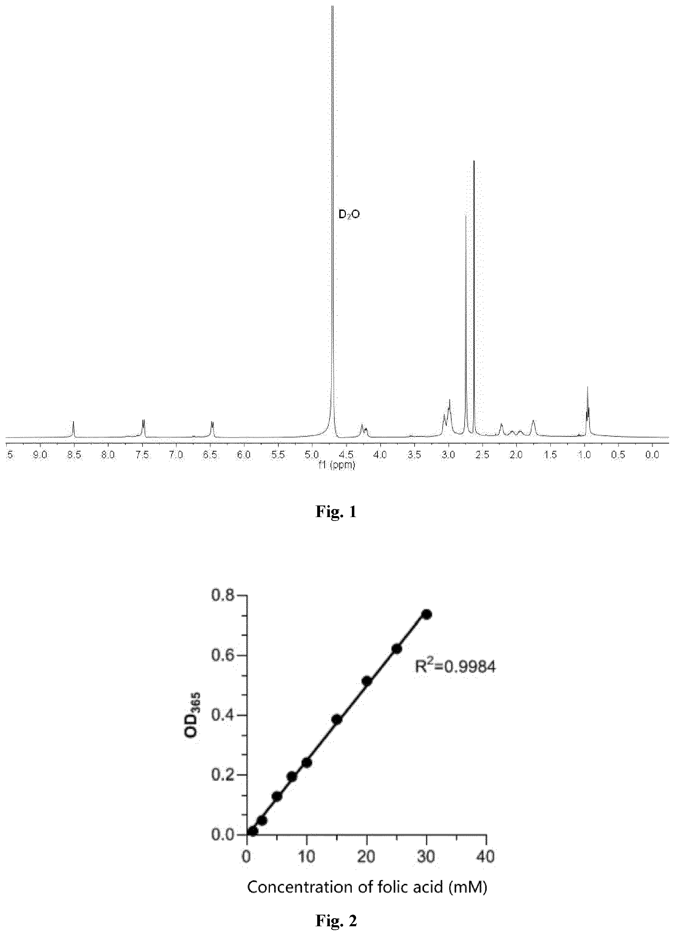

Folic acid itself is insoluble in water. Upon linking with OVA, the folate-OVA complex is easily soluble in water due to the good water solubility of the protein, suggesting that folate was successfully linked to OVA. **Fig. 1** provides the ¹HNMR of the obtained folate-OVA complex, wherein the signals δ7.45 and δ6.45 are the signal peaks of a group of symmetrical "AA'BB"' spin systems. Thus, it can be determined that said signals are the two groups of proton signal peaks of the para-disubstituted benzene ring in the folic acid molecule, suggesting that the molecule of the product contains the structute of folate. The structue of the product is further determined to be folate-OVA. The OD₃₆₅ₙₘ of the obtained folate complex was measured by an ultraviolet spectrophotometer. **Fig. 2** provides the standard curve to quantify the degree of folate modification. As shown in Table 1, the obtained folate-OVA has a degree of folate modification of about 8.66. The degree of folate modification means the ratio of folate in the folate complex to the folate complex.

Folate-coagulation factor VIII (FA-FVIII) was synthesized as another example of folate-modified protein complex. FVIII stock solution (20 IU/mL, obtained from Shanghai RAAS) was half-diluted with 0.1 M carbonate buffer solution (pH=9.5). The 50-fold equivalents of folic acid and 60-fold equivalents of EDC (both dissolved in 0.1 M carbonate buffer) were added. Within 15 minutes after the addition, 2 M hydrochloric acid solution was added dropwise to adjust the pH of the reaction solution to 6.6. The reaction was shaken at room temperature overnight, then dialyzed for 72h, to obtain the product. As shown in **Table 1,** the calculated degree of folate modification of FA-FVIII is about 8.03.

### Example 2: Preparation of folate-monoclonal antibody complex

Folate-adalimumab complex (FA-Adalimumab) and folate-trastuzumab complex (FA-Trastuzumab) were prepared as examples of folate-monoclonal antibody complexes. A PBS solution containing 1 mg/mL each mAbs was prepared respectively, and tris(2-carboxyethyl)phosphine and EDTA were added to a concentrations of 10 mM and 5 mM, respectively. The reaction was shaken at room temperature for 90 minutes, and then was mixed with 15 equivalents of folate-PEG₄₀₀-Mal (Folate-PEG₄₀₀-Maleimide, purchased from Shanghai Yisheng Biotechnology Co., Ltd.). The reaction was shaked at room temperature for 20 minutes. In order to block the unreacted sulfhydryl groups, the solution was further reacted with 15 equivalents of maleimide at room temperature for 20 minutes under shaking. Subsequently, the reaction solution was dialyzed aginst PBS for 72h to remove small molecules from the system, to obtain folate-monoclonal antibody complex solution. Table 1 provides the degree of folate modification in the folate-mAb complexs.

**Table 1: The results of the degree of folate modification in each folate-modified biomacromolecule prepared in Examples 1-2**

| sample | concentration of sample | OD₃₆₅nm | Concentration of folate in the complex | Degree of Modification | |
|---|---|---|---|---|---|
| Folate-OVA | 1 mM | 0.41 | 8.66 mM | 8.66 | (per molecule) |
| Folate-FVIII | 1 IU/l | 0.35 | 14.20 IU/I | 14.20 | (per IU) |
| Folate-Adalimumab | 2 mM | 0.40 | 16.068 mM | 8.03 | (per molecule) |
| Folate-Trastuzumab | 2 mM | 0.53 | 21.35 mM | 10.68 | (per molecule) |

### Example 3: Preparation of folate-contrast agent complexs

Folate-PEG₂₀₀₀-Cy5 (i.e., FA-PEG₂₀₀₀-Cy5) was prepared as an example of folate-contrast agent complexs. Folic acid (0.57g, 1.28mmol), NHS (0.37g, 2.8mmol) and DCC (0.6g, 2.8mmol) were added into a 250 mL round-bottom flask, and then DMSO (75mL) was added. Reaction was performed under nitrogen protection at 25°C. After the substantial activation of folic acid monitored by TLC, the reaction was passed through a 0.22 µm organic filter membrane, and NH₂-PEG₂₀₀₀-Cy5 (3g, 1.15mmol, purchased from Xi'an Ruixi Biological Technology Co., Ltd) and TEA (350 µl) were added. After 2h, the reaction was complete as monitored by HPLC. The reaction solution was purified by G50 silica gel column (eluent: 0.01M PBS, pH 8.0) to obtain FA-PEG₂₀₀₀-Cy5.

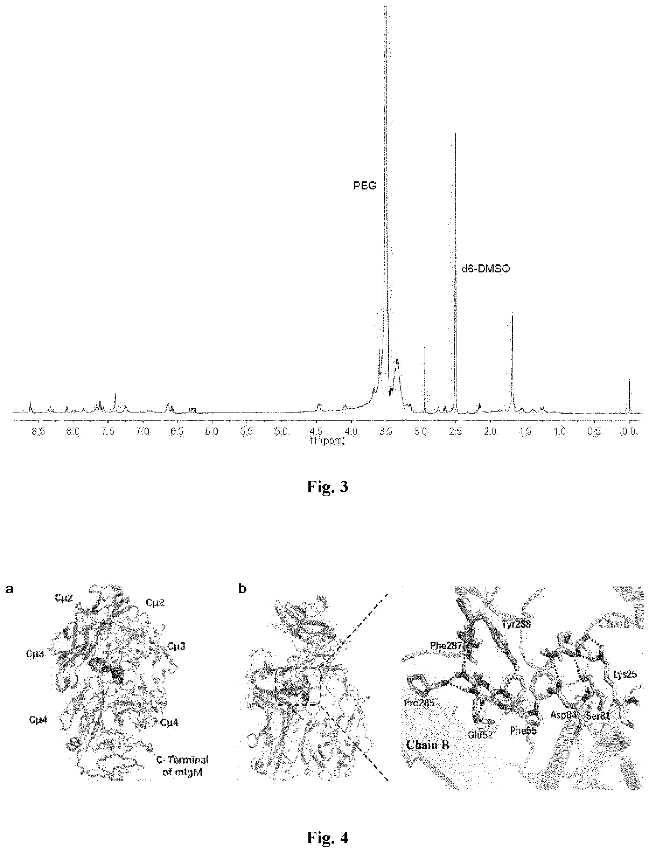

Folic acid itself is insoluble in water, and the introduction of PEG₂₀₀₀ improves the water solubility of folic acid. **Fig. 3** provides the ¹HNMR of the obtained FA-PEG₂₀₀₀-Cy5, wherein the signals δ7.65 and δ6.65 are the signal peaks of a group of symmetrical "AA'BB"' spin systems. Thus, it can be determined that said signals are the two groups of proton signal peaks of the para-disubstituted benzene ring in the folic acid molecule, suggesting that the molecule of the product contains the structute of folate. The signal δ3.51 is a typical signal peak of PEG, further determining that the structure of the product is FA-PEG₂₀₀₀-Cy5.

### Example 4: Specific binding of folate to mIgM on the surface of B-cell

The binding mode of folate in the murine IgM Fc domain was investigated by homology modeling and molecular docking. Molecular docking studies were performed with Schrodinger2015 software to investigate the binding mode of folate in IgM Fc. **Fig. 4a** shows the binding mode of folate to murine mIgM. It can be seen that the binding pocket of folate to mIgM is located between Cµ3 and Cµ4 of the Fc fragment. **Fig. 4b** further provides an analysis on the binding force of folate to murine IgM monomers, wherein the binding pocket of folate is located at the junction of the two heavy chains (chain A and chain B) of the IgM monomer; the pteridine fragment in the folate structure may form hydrogen bonds with Phe287, Tyr288 and Pro285 in chain A and Glu52 in chain B; Phe55 in chain B forms a π-π interaction with the benzene fragment of folate; Lys25 in chain B forms an ionic interaction with the carboxyl group. The predicted binding energy between folate and Fc fragment of IgM is -10.929 kcal/mol, and the affinity reaches a level of 10⁻⁸ mol.

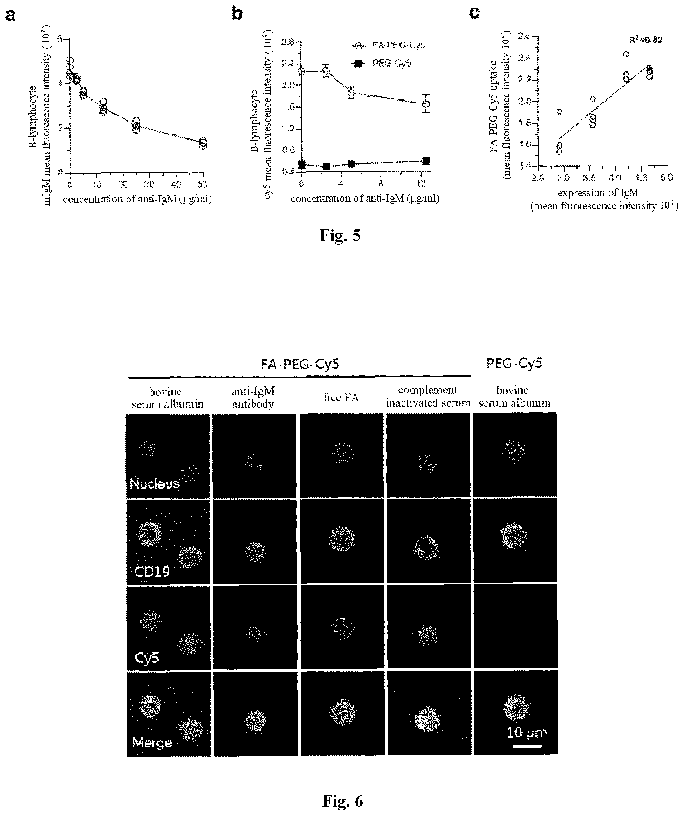

The binding of folate to mIgM was investigated with mIgM-positively-expressed spleen B-cell. BALB/c mouse spleen lymphocytes were extracted from mouse spleen lymphocyte isolates (purchased from Solarbo Technology Co., Ltd.), counted to 2×10⁶/mL, and plated on a 24-well culture plate (1mL/well). Anti-IgM (ab97230, 1mg/mL) was added to each well to the working concentrations of 0, 2.5, 5, 12.5 µg/mL. After being incubated at 37°C for 24h, the cells were divided into FA-PEG-Cy5 group and PEG-Cy5 group (n=3-4). 10 µL of FA-PEG-Cy5 (0.1 mM, prepared according to Example 3) or PEG-Cy5 (0.1 mM, purchased from Xi'an Ruixi Biological Co., Ltd.) were added, and the uptake was performed at 37°C for 0.5h. After centrifugation and washing of the cells, FITC-IgM and CD19-PE were added and the mixture was incubated at 4°C for 1h to label mIgM and CD19 on the cell surface. The expression level of mIgM in mouse spleen B-cell and the uptake of FA-PEG-Cy5 by cell were measured with flow cytometry. **Fig. 5** provides the results of flow cytometric analysis of the binding of folate to mIgM. The results in **Fig. 5a** show that the mouse spleen B-cell mIgM can be effectively reduced with the increase of the concentrations of anti-IgM antibodies. The results in **Fig. 5b** show that the uptake of FA-PEG-Cy5 gradually decreased with the increase of anti-IgM concentrations, while the uptake of PEG-Cy5 does not vary much. As shown in **Fig. 5c****,** by linearly fitting the expression level of mIgM to the uptake of FA-PEG-Cy5, the correlation coefficient R² is 0.82, indicating that the uptake ability of FA-PEG-Cy5 is positively correlated with the expression level of mIgM.

The binding of folate to mIgM was further investigated by a confocal microscope. The extracted BALB/c mouse spleen lymphocytes were counted to 2×10⁶/mL. Anti-IgM antibodies (anti-mouse IgM, ab97230, 10 µg/mL), free folic acid molecule (0.5M), complement inactivated serum (serum was heat-treated at 56°C for 30min) or bovine serum albumin (5%) was added to the cells respectively, and the mixture was incubated at room temperature for 1h. Then, the mixture was co-incubated with FA-PEG₂₀₀₀-Cy5 (prepared according to Example 3) at room temperature for 1h. Similarly, the cells were incubated with bovine serum albumin (5%) at room temperature for 1h and then co-incubated with PEG₂₀₀₀-Cy5 (purchased from Xi'an Ruixi Biological Co., Ltd.) for 1h at room temperature. B-cells were screened for CD19 marker, and the binding of folate to mIgM was observed by confocal laser microscopy. **Fig. 6** provides confocal laser micrographs (Carl Zeiss LSM710, Germany) of folate binded to mIgM, showing that the binding of folate (FA-PEG₂₀₀₀-Cy5) to B-cell is much higher than that of PEG₂₀₀₀-Cy5 and can be inhibited by anti-IgM antibodies and free folic acid molecules, independent of serum complement proteins, i.e., independent of complement receptors. The above results show that folate can specifically bind and target to mIgM of B-cell.

### Example 5: Evaluation of targeting of folate to spleen marginal region B-cell

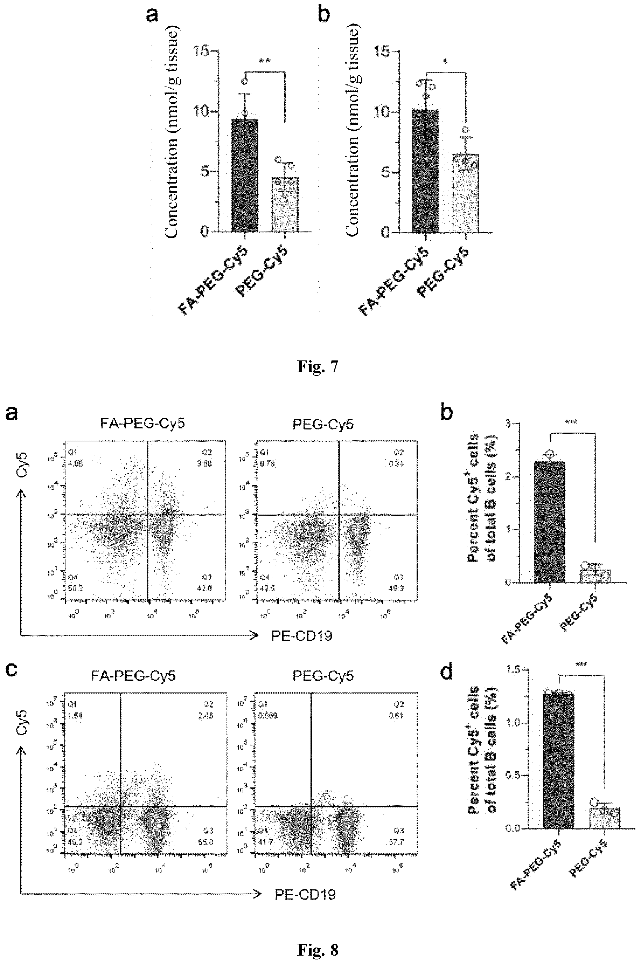

BALB/c mice (male, 6 weeks old, Slac) were injected with folate-contrast agent complex FA-PEG₂₀₀₀-Cy5 (prepared according to Example 3) or PEG₂₀₀₀-Cy5 (as a control) via tail vein in 100nmol/mouse, with three in each group. Mice were sacrificed at 1h and 4h respectively, and spleens were isolated. The tissue was homogenated. According to the fluorescence standard curve of FA-PEG₂₀₀₀-Cy5 or PEG₂₀₀₀-Cy5, the folate concentration in the spleen homogenate was measured with a fluorence microplate reader, and the data were shown in **Fig. 7** (**a:** 1 hour; **b:** 4 hours, ^{∗}p<0.05, ^{∗∗}p<0.01, ^{∗∗∗}p<0.001, one-way ANOVA). **Fig. 7** shows that: 1 hour after injection, the spleen accumulations of FA-PEG₂₀₀₀-Cy5 and PEG₂₀₀₀-Cy5 were 9.35±2.10 nmol/g tissue and 4.57±1.19 nmol/g tissue, respectively; 4 hours after injection, the spleen accumulations of FA-PEG₂₀₀₀-Cy5 and PEG₂₀₀₀-Cy5 were 10.20±2.44 nmol/g tissue and 7.07±1.44 nmol/g tissue, respectively. The accumulation of FA-PEG₂₀₀₀-Cy5 in the spleen was significantly higher than that of the control PEG₂₀₀₀-Cy5, suggesting the spleen-targeting ability of FA-PEG₂₀₀₀-Cy5.

In addition, the spleens of mice sacrificed at 1h or 4h were collected, and the spleen lymphocytes were isolated for flow cytometric analysis (CytoFlex S, Beckman). **Fig. 8** shows the flow sorting graphs (**a:** 1h; c: 4h) and statistical analysis results (**b:** 1h; **d:** 4h) of the spleen lymphocytes of mice sacrificed at 1h or 4h after administration of FA-PEG₂₀₀₀-Cy5 or PEG₂₀₀₀-Cy5, respectively. The results show that folate has a significant B-cell targeting ability.

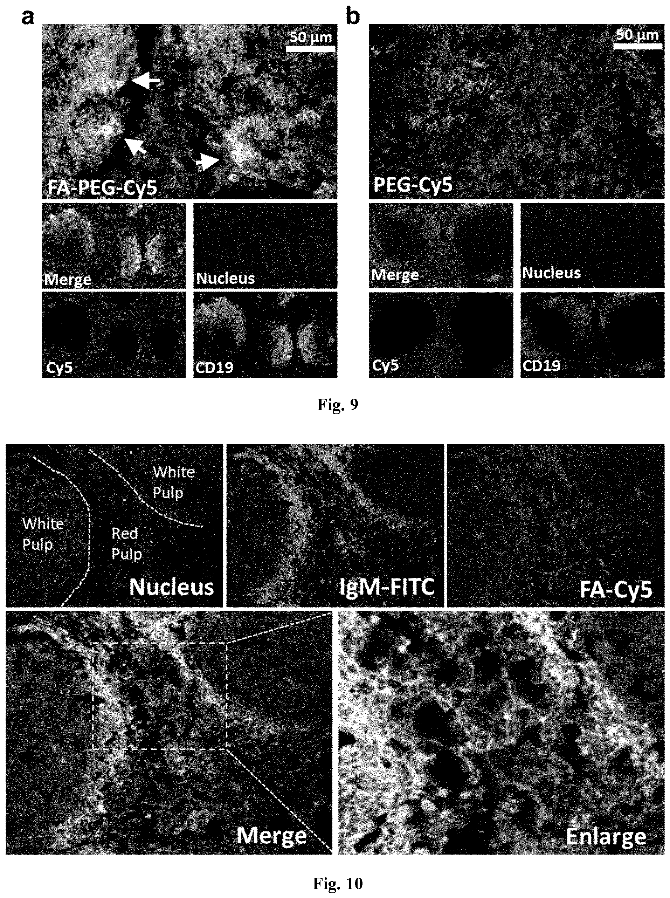

The spleens from the mice sacrificed at 1h were subjected to cryosection analysis. **Fig. 9** shows the immunofluorescence photographs of the distribution of FA-PEG₂₀₀₀-Cy5 (a) and PEG₂₀₀₀-Cy5 (b) in the spleens, wherein the blue is the cell nucleus labeled with DAPI, the green is the B-cells labeled with CD19-PE antibody (553786, BD Pharmingen), the red is Cy5, and the yellow is the overlapping color of green and red. The results showed that folate was mainly distributed in the marginal area of the spleen, and significantly overlap the marginal B-cell. That is, folate targets the marginal B-cell of the spleen.

Since the splenic marginal B-cell highly expresses mIgM, the marginal B-cell was labeled with anti-IgM-FITC antibody (148445, Jackson). **Fig. 10** provides the immunofluorescence photographs of the distribution of FA-PEG₂₀₀₀-Cy5 in the splenic marginal B-cell, wherein the blue is cell nucleus labeled with DAPI, the green is the marginal B-cell labeled with anti-IgM-FITC, the red is Cy5, and the yellow is the overlapping color of green and red. The results showed that folate significantly overlap the marginal B-cell. That is, folate targets the marginal B-cell of the spleen.

### Example 6: The role of folate modification in mediating humoral immunosuppression and in reducing Anti-Drug Antibodies of protein biomacromolecular drugs

The role of folate modification in mediating humoral immunosuppression and in reducing Anti-Drug Antibodies (ADA) of biomacromolecular drugs was investragated by using ovalbumin (OVA) as a model protein. OVA has strong immunogenicity and can induce the body to produce an immune response. Repeated stimulation of mice by OVA can cause the mice to produce lots of specific antibodies. The BALB/c female mice (5 weeks old) were randomly divided into FA-OVA (folate-OVA complex, Example 1) group, OVA group and physiological saline control group, with 6 mice in each group. FA-OVA, OVA and physiological saline were administered to mice via tail vein injection at a dose of 10 µg/mouse, once a week, three times in total.

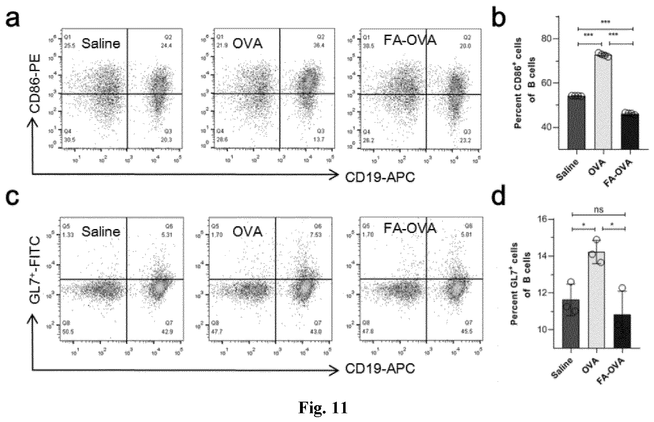

One week after the last administration, the spleen lymphocyte suspension was collected and subjected to flow cytometry for analysis of proliferation and differentiation of splenic B lymphocytes. CD86 was used as a marker for cell proliferation, and GL-7 was used as germinal center B-cell surface marker. **Figures 11a** and **11b** provide the CD86 flow sorting graphs and statistical analysis results of mouse spleen lymphocytes, respectively. **Figures 11c** and **11d** provide the GL7 flow sorting graphs and statistical analysis results of mouse spleen lymphocytes, respectively. It can be seen from **Fig. 11** that the B-cell in the OVA group mice proliferated significantly and differentiated into the germinal center B-cell, while the proliferation of B-cell and the differentiation into germinal center B-cell were significantly inhibited in the FA-OVA group mice, suggesting the efficacy of folate modification in inhibiting B-cell proliferation and differentiation, which results in B-cell anergy.

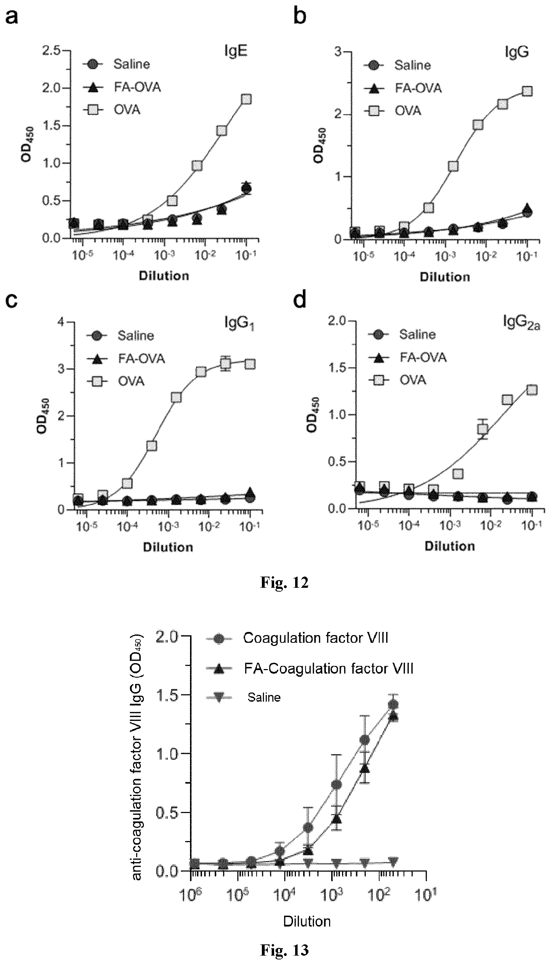

One week after the last administration, the serum of the mice was collected and was measured by ELISA for the expression of OVA-specific antibodies in the serum. The results are shown in **Fig. 12****,** wherein **Figures 12a, 12b, 12c** and **12d** show the results of OVA-specific IgE, IgG, IgGi and IgG₂ₐ, respectively. The data showed that repeated injections of OVA induced the body to produce a large amount of OVA-specific antibodies, while the expression of antibodies in FA-OVA group mice was significantly inhibited, which was comparable to the level in the physiological saline control group. This shows that the folate modification can significantly reduce the immunogenicity of antigenic substances, e.g., OVA, effectively inhibit humoral immunity, and reduce the production of Anti-Drug Antibodies of biomacromolecular protein drugs.

In addition, the whole blood of the mice was collected one week after the last administration for blood routine analysis. The results showed that there were no obvious abnormalities in the blood routine indicators of the mice in each group, indicating that the folate modification did not produce obvious side effects. The data are shown in Table 2 below.

**Table 2:**

| | FA-OVA | OVA | physiological saline |
|---|---|---|---|
| WBC (10^{∗}9/L) | 3.77±0.42 | 3.43±0.51 | 4.35±0.70 |
| RBC (10^{∗}12/L) | 9.96±0.89 | 10.55±1.13 | 10.56±0.40 |
| HGB (g/L) | 161.50±9.26 | 169.25±13.45 | 172.00±4.00 |
| HCT (%) | 49.83±4.53 | 52.15±5.97 | 53.37±1.55 |
| MCV(fL) | 50.075±1.37 | 49.43±0.43 | 50.53±0.45 |
| MCH (pg) | 16.28±0.59 | 16.08±0.67 | 16.30±0.20 |
| MCHC (g/L) | 325.00±12.35 | 325.75±16.50 | 322.33±1.53 |
| PLT (10^{∗}9/L) | 586.50±59.29 | 635.00±36.59 | 681.00±82.31 |
| RDW-CV (%) | 14.78±0.50 | 14.63±0.33 | 15.10±0.17 |
| PDW (fL) | 5.58±0.096 | 5.53±0.15 | 5.57±0.31 |
| MPV (fL) | 7.05±0.058 | 7.10±0.14 | 7.067±0.21 |
| P-LCR (%) | 4.35±1.01 | 5.15±1.48 | 5.37±1.06 |
| PCT (%) | 0.41±0.042 | 0.45±0.016 | 0.49±0.067 |
| NRBC (10*9/L) | 0.015±0.013 | 0.025±0.013 | 0.030±0.020 |
| NEYT (10*9/L) | 0.94±0.23 | 0.80±0.21 | 0.82±0.27 |
| LYMPH (10*9/L) | 2.78±0.19 | 2.58±0.29 | 3.42±0.52 |
| MONO (10*9/L) | 0.035±0.0058 | 0.035±0.017 | 0.033±0.011 |
| BASO (10*9/L) | 0 | 0 | 0 |

The role of folate modification in reducing Anti-Drug Antibodies (ADAs) of protein biomacromolecular drugs was investragated by using coagulation factor VIII (FVIII) as a model protein. The BALB/c female mice (5 weeks old) were randomly divided into FVIII group, FA-FVIII group and physiological saline control group, with 5 mice in each group. FVIII, FA-FVIII (prepared according to Example 1) and physiological saline were administered to mice via tail vein injection at a dose of 0.5 IU/mouse, once a week, three times in total. One week after the last administration, the serum of the mice was collected and was measured by ELISA for the expression of FVIII-specific antibodies in the serum. The results in **Fig. 13** show that the antibody level in the FA-FVIII group was significantly lower than that in the FVIII group, suggesting that folate modification effectively reduces the production of Anti-Drug Antibodies of protein drugs.

### Example 7: Effect of folic acid in mediating humoral immunosuppression

The BALB/c female mice (5 weeks old) were randomly divided into 4 groups, with 6 mice in each group, and were treated as follows: (1) FA(ig) group: each mouse was administered with 3mg free folic acid by gavage every day for two weeks; (2) FA(ig)+OVA group: each mouse was administered with 3mg free folic acid by gavage every day for two weeks, and was administered with OVA via tail vein injection on day 0 and day 7 respectively (10 µg/mouse); (3) OVA group: each mouse was administered with OVA via tail vein injection on days 0 and day 7 respectively (10 µg/mouse); (4) Physiological saline group: each mouse was administered with physiological saline via tail vein injection on day 0 and day 7 respectively. Then, on days 14, 21 and 28, all mice were administered with 100µl mixed solution of OVA and KLH (hemocyanin) in water (100 ug/mL OVA and 100 ug/mL KLH). The serum was collected before the administration of the mixed solution on days 14, 21 and 28 and on day 35. The specific antibodies in the serum were detected by ELISA.

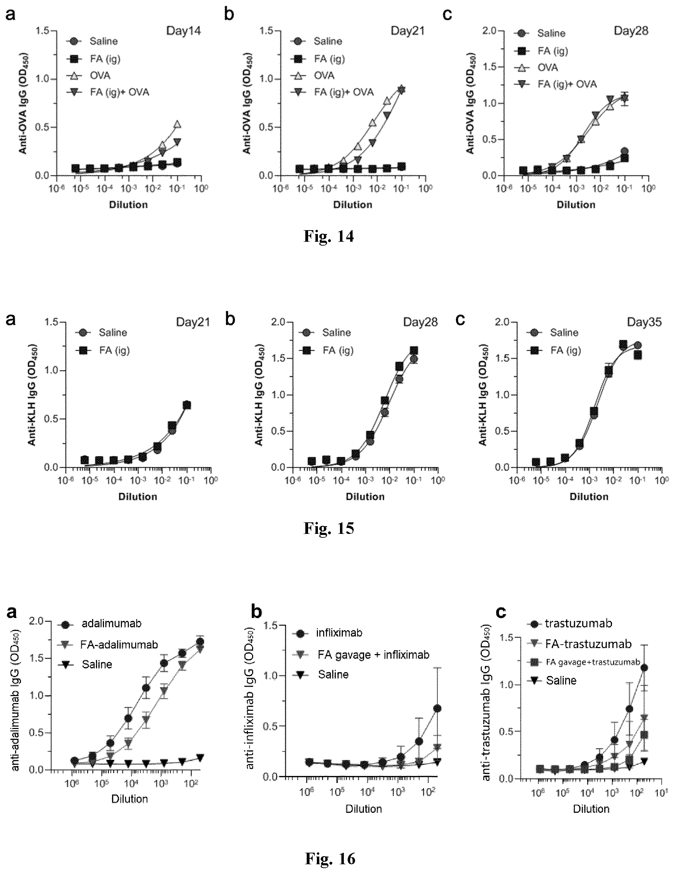

**Fig. 14** shows the levels of OVA-specific antibodies in mouse serum on day 14 **(a),** day 21 **(b)** and day 28 **(c),** respectively. The results showed that, on days 14 and 21, the level of OVA-specific IgG in the serum of the FA(ig)+OVA group mice was significantly lower than that in the OVA group, suggesting that folate exerts an effect of humoral immunosuppression and the combination of FA and OVA can reduce the production of OVA-specific antibodies. On day 28, the level of OVA-specific IgG in the serum of the FA(ig)+OVA group mice was comparable to that in the OVA group, suggesting that the immunosuppressive effect of FA is reversible and non-persistent. The immune function of the body recovered when the administration of FA is discontinued.

**Fig. 15** shows the levels of KLH-specific antibodies in mouse serum on day 21 **(a),** day 28 **(b)** and day 35 **(c),** respectively. The results showed that the levels of KLH-specific antibodies in FA(ig) group and physiological saline group were similar, and both increased with the increase of injection times. The results also confirmed that the immune function of the body recovered when the administration of FA is discontinued.

### Example 8: Efficacy of folic acid and folate modification in reducing the production of Anti-Drug Antibodies of antibodies biomacromolecular drugs

Adalimumab and infliximab are humanized monoclonal antibodies against human tumor necrosis factor (TNF), and trastuzumab is a humanized monoclonal antibody against human epidermal growth factor receptor-2 (HER2). Their repeated injections are very likely to induce the body to produce antibodies against said mAbs, affecting the efficacy of the mAbs. The efficancy of folic acid and folate modification in reducing the Anti-Drug Antibodies of antibodies drugs was investigated with infliximab and trastuzumab as model mAbs.

The BALB/c female mice (6 weeks old) were randomly divided into Adalimumab group, FA-Adalimumab group (prepared according to Example 2) and physiological saline group, with 5 mice in each group. For the Adalimumab group and the FA-Adalimumab group, a dose of 30 µg antibody/mouse was administrated by subcutaneous injection on days 0, 7 and 14. For the physiological saline group, the mice were subcutaneously injected with physiological saline on days 0, 7 and 14. One week after the last administration (day 21), the serum was collected from the mice, and was measured by ELISA for the antibodies against Adalimumab. The results are shown in **Fig. 16a****.** The results showed that the level of antibodies in the FA-Adalimumab group was significantly lower than that in the Adalimumab group, suggesting that folate modification effectively reduced the production of Anti-Drug Antibodies of the antibody drugs.

The BALB/c female mice (6 weeks old) were randomly divided into infliximab group, FA gavage + infliximab group and physiological saline group, with 5 mice in each group. For the infliximab group, a dose of 60 µg antibody/mouse was administrated via tail vein injection on days 0, 7 and 14. For the FA gavage + infliximab group, each mouse was administered with 3 mg free folic acid by gavage per day for two weeks, and infliximab was administered via tail vein injection at a dose of 60 µg antibody/mouse on days 0, 7 and 14. For the physiological saline group, the mice were injected with physiological saline via tail vein on days 0, 7 and 14. One week after the last administration (day 21), the serum was collected from the mice, and was measured by ELISA for the antibodies against infliximab. The results are shown in **Fig. 16b****.** The results showed that the level of antibodies in the FA gavage + infliximab group was significantly lower than that in the infliximab group, suggesting that FA effectively reduced the production of Anti-Drug Antibodies of the antibody drugs.

The BALB/c female mice (6 weeks old) were randomly divided into trastuzumab group, folate-trastuzumab group (obtained according to Example 2), FA gavage + trastuzumab group and physiological saline group, with 5 mice in each group. For the trastuzumab group and the folate-trastuzumab group, a dose of 60 µg antibody/mouse was administrated via tail vein injection on days 0, 7 and 14. For the FA gavage + trastuzumab group, each mouse was administered by gavage with 3 mg free folic acid per day for two weeks, and trastuzumab was administered via tail vein injection at a dose of 60 µg antibody/mouse on days 0, 7 and 14. For the physiological saline group, the mice were injected with physiological saline via tail vein on days 0, 7 and 14. One week after the last administration (day 21), the serum was collected from the mice, and was measured by ELISA for the antibodies against trastuzumab. The results are shown in **Fig. 16c****.** The results showed that the levels of antibodies in the FA gavage + trastuzumab group and in the folate-trastuzumab group were significantly lower than that in the trastuzumab group, suggesting that FA and folate modification effectively reduced the production of Anti-Drug Antibodies of the antibody drugs.

### Example 9: Efficacy of folic acid in reducing the production of antibodies aginst drug delivery systems

The efficacy of folic acid in reducing the production of antibodies aginst drug delivery systems was investigated with liposome as an example.

Liposomes (sLip) were prepared firstly. HSPC, Chol and mPEG₂₀₀₀-DSPE (all purchased from AVT (Shanghai) Pharmaceutical Tech Co., Ltd.) were weighted in a molar ratio of 52:43:5. The film materials were dissolved in 5 mL chloroform, and the mixture was subjected to rotary evaporation under reduced pressure to remove chloroform, to obtain a uniform lipid film. Residual organic solvent was removed by vacuum drying overnight. Shaking in a water bath at 60°C was performed until the liposome membrane is completely hydrated to obtain white liposome suspension. The liposome suspension went through 200 and 100 nuclear pore membrane by a micro-extruder sequentially, to obtain a liquid with pale blue opalescence, *i.e.*, a liposome (sLip) solution.

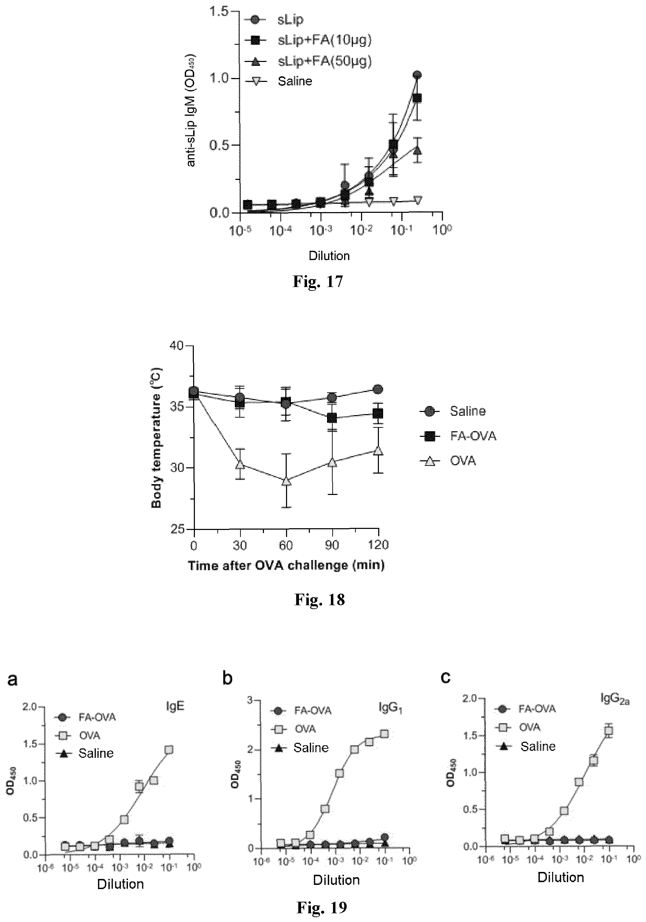

The BALB/c female mice (6 weeks old) were randomly divided into sLip group, sLip+FA (10µg) group, sLip+FA (50µg) group and physiological saline group, with 4 mice in each group. For the sLip group, the liposome solution (5 mg liposome/kg mouse body weight) was administered in a single dose via tail vein. For sLip+FA(10µg) group, a mixed solution of liposome (5 mg liposome/kg mouse body weight) and 10µg FA was administered in a single dose via tail vein. For sLip+FA(50µg) group, a mixed solution of liposome (5 mg liposome/kg mouse body weight) and 50ug FA was administered in a single dose via tail vein. For the physiological saline group, each mouse was injected with the same volume of physiological saline via tail vein. Serum was collected from all mice 5 days after administration, and was measured by ELISA for the concentrations of the antibodies against sLip. The results are shown in **Fig. 17****.** The results show that the combination of folic acid and liposomes can effectively reduce the production of the antibodies against liposomes, and the effect increases as the concentration of folic acid increases.

### Example 10: Efficacy of folate modification in the prevention of anaphylactic shock

The BALB/c female mice (5 weeks old) were randomly divided into FA-OVA group, OVA group and physiological saline group, with 10 mice in each group. FA-OVA or OVA or physiological saline of an equal volume was administered to the mice via tail vein injection at a dose of 10µg OVA/mouse, once a week for a total of three times. One week after the last administration, each mouse was injected with 10µg OVA (allergen) via tail vein. Changes in body temperature of the mice within 2 hours after OVA stimulation were recorded **(****Fig. 18****).** In the meanwhile, the serum was collected from the mice, and was measured by ELISA for the expression of OVA-specific antibodies in the serum **(****Fig. 19). Fig. 18** shows that the body temperature of the FA-OVA group mice did not change significantly, which was substantially comprarble to that in the physiological saline group. However, the OVA group mice experienced severe anaphylactic shock, their body temperature dropped significantly, and two mice died. **Fig. 19** shows that the FA-OVA group is substantially comparable to the physiological saline group, while the OVA group mice produced a large number of OVA-specific antibodies, suggesting that folate modification can significantly reduce the production of the antibodies caused by OVA. Therefore, folate modification can effectively prevent anaphylactic shock induced by allergens.

### Example 11: Efficacy of folate modification in the treatment of anaphylactic shock

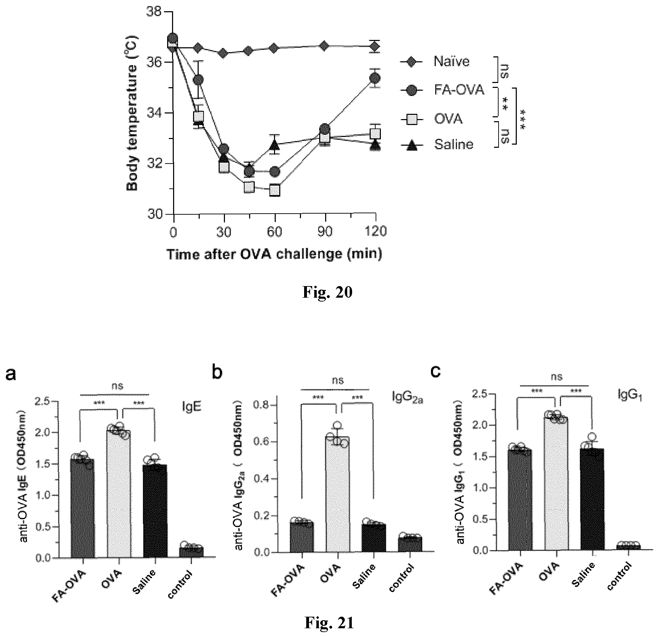

The BALB/c female mice (5 weeks old) were subcutaneously injected with 10µg OVA for sensitization, once a week, three times in total. One week after the last sensitization, the OVA-sensitized mice were injected with FA-OVA, OVA and physiological saline via tail vein at a dose of 4µg/mouse, once every two days for three times in total. After the last administration, the mice were injected with 25µg OVA (allergen) via tail vein. Changes in body temperature of the mice within 2 hours after the injection were recorded **(****Fig. 20****).** In the meanwhile, the serum was collected from the mice and was measured by ELISA for the level of the OVA-specific antibodies in the serum (Fig. 21). The mice without OVA sensitization but stimulated by tail vein injection of OVA served as the Naive control group.

**Fig. 20** shows the body temperature change curves of the sensitized mice which was treated with FA-OVA, OVA and physiological saline respectively and then was challenged with OVA. It can be seen that FA-OVA treatment showed a desensitizing effect, and the decrease in body temperature of the mice was controlled and returned to normal within 2 hours.

**Fig. 21** shows the levels of serum OVA-specific antibodies in the sensitized mice which was treated with FA-OVA, OVA and physiological saline respectively and then was challenged with OVA. It can be seen that the expressions of OVA-specific antibodies IgE **(a),** IgG₁ **(b)** and IgG₂ₐ **(c)** in the FA-OVA group were comparable to those in the physiological saline group and significantly decreased compared with those in the OVA group, suggesting the efficacy of folate modification in the treatment of antigen allergic diseases.

### Example 12: Efficacy of folate modification in autoimmune encephalomyelitis

The efficacy of folate modification in autoimmune encephalomyelitis was investigated using an experimental autoimmune encephalomyelitis (EAE) model. The model was established as follows: the animals were injected with whole protein or protein fragment (e.g., PLP polypeptide) that induces nerve cell myelin sheath and peripheral protein, causing inflammation and demyelination symptoms and inducing the autoimmune response of the animal; thereby the injected self-proteins are regarded as foreign objects and cause the immune system to attack the self myelin sheath.

The female C57BL6 mice were intravenously injected with PLP polypeptide (amino acid sequence: HSLGKWLGHPDKF) or folate-PLP complex (FA-PLP) at a dose of 10µg/mouse, once a week for three times in total. One week later, the emulsion of PLP polypeptide in Freund's complete adjuvant was prepared, the concentration of PLP being 1 µg/µL. Four points were set up on both sides of the back of the mouse, and 0.05mL PLP emulsion was injected subcutaneously at each point to induce inflammation. 2 and 24 hours after PLP injection, 150 ng pertussis toxin was injected intraperitoneally. The incidence in the mice was observed every day, and the mice were scored according to the 5-point scoring standard (Kono method). The incidence scoring curve was ploted. The results show that the modification of PLP by folate causes the body's immune tolerance to PLP and contributes to reducing the inflammatory response.

### Example 13: Efficacy of folate modification in bone marrow transplant rejection

The CD45.1 mice donated bone marrow and the CD45.2 mice received bone marrow transplantation. 7 days before and 1 day after bone marrow transplantation, the CD45.2 mice were injected with FA-Dby (folate-modified Dby polypeptide; the amino acid sequence of Dby is NAGFNSNRANSSRSS) or FA+Dby (a physical mixture of folic acid and Dby in water) via tail vein at a dose of 10µg/mouse. One day beofre bone marrow transplantation, each CD45.2 mouse received 200 cGy low-dose radiation. The donor mice were sacrificed, the femur and tibia were cut on an ultra-clean bench, and the cavity was purged repeatedly. The bone marrow cells were extracted, and washed with cPBS. The Recipient mice were injected intravenously with 5×10⁶ cells. After bone marrow transplantation, blood was collected from the mice once a week, and the chimerism of lymphocytes was measured by flow cytometry. Purebred CD45.1 mice were used as the control. The measurement indicators are CD45.1/CD45.2 and CD45.1/CD90.2.

### Example 14: Efficacy of folate-human serum albumin conjugate in reducing the immunogenicity of biomacromolecule and drug delivery system

The efficacy of folate conjugates in reducing the immunogenicity of biomacromolecule (OVA, trastuzumab) and drug delivery system (sLip) was inventigated with folate-human serum albumin (FA-HSA, purchased from Xi'an Ruixi Biological Technology Co., Ltd) as a representative of folate conjugates.

The BALB/c female mice (5 weeks old) were randomly divided into OVA group, FA-HSA + OVA group and physiological saline control group, with 6 mice in each group. The following treatments were performed respectively: (1) OVA group: each mouse was injected with 10µg OVA via tail vein on days 0, 7 and 14; (2) FA-HSA + OVA group: each mouse was injected with a mixed solution of 10µg OVA and 10µg FA-HAS via tail vein on days 0, 7 and 14; (3) Physiological saline group: each mouse was injected with the same volume of physiological saline via tail vein on days 0, 7 and 14. Serum was collected from all mice on day 21, and was measured by ELISA for the OVA-specific antibody (IgG) titer.

The above experiment was repeated with trastuzumab instead of OVA, adjusting the dose to 60µg antibody/mouse.

The BALB/c female mice (6 weeks old) were randomly divided into sLip group (prepared according to Example 9), sLip + FA-HSA group and physiological saline group, with 6 mice in each group. For the sLip group, the mice were injected with liposome solution (5 mg liposome/kg mouse body weight) in a single dose via tail vein. For the sLip + FA-HSA group, the mice were injected with a mixed solution of liposome (5 mg liposome/kg mouse body weight) and 10µg FA-HSA in a single dose via tail vein. For the physiological saline group, the mouse was injected with the same volume of physiological saline via tail vein. Five days after administration, the serum was collected from all mice, and was measured by ELISA for the antibody (IgM) titer against sLip in the serum.

### Example 15: Efficacy of folate conjugates in reducing the immunogenicity of biomacromolecule and drug delivery system

Similarly to Example 14, the efficacy of folate-polyethylene glycol_{40kDa} (FA-PEG_{40kDa}, purchased from Xi'an Ruixi Biological Technology Co., Ltd) in reducing the immunogenicity of biomacromolecule (OVA, trastuzumab) and drug delivery system (sLip) is inventigated with FA-PEG_{40kDa} instead of FA-HAS. FA-PEG_{40KDa} is administrated at a dose of 100 nM/mouse.

### Formulation Examples

### Example A

Injectable solutions containing the agent according to the invention can be prepared in a conventional manner:

| Ingredients | Amount (per mL) |
|---|---|
| Agent according to the invention | 5.0 mg |
| Polyethylene glycol 400 | 150.0 mg |
| Acetic acid | qs to pH 5.0 |
| Water for Injection | add to 1.0 mL |

The agent according to the invention is dissolved in a mixture of polyethylene glycol 400 and water for injection (part). Acetic acid is added to adjust pH to 5.0. The volume is made up to 1.0 mL by adding the remaining amount of water. The solution is filtered, filled into vials in appropriate overdose, and sterilized.

### Example B

Oral tablets containing the following ingredients can be prepared in a conventional manner:

| Ingredients | per tablet |
|---|---|
| Agent according to the invention | 10 mg |
| Corn starch | 80 mg |
| Lactose | 95 mg |
| Magnesium stearate | 5 mg |

The above ingredients are mixed homogeneously and are compressed into tablets.

### Example C

Oral capsules containing the following ingredients can be prepared in a conventional manner:

| Ingredients | per capsule |
|---|---|
| Agent according to the invention | 10.0 mg |
| Lactose | 95.0 mg |
| Corn starch | 20.0 mg |
| Talc | 5.0 mg |

The ingredients are sieved, blended and filled into capsule shells.

### Example D

Aerosol containing the following ingredients can be produced in a conventional manner:

| Ingredients | Content (per 100mL) |
|---|---|
| Agent according to the invention | 80.0 mg |
| Oleic acid | Appropriate amount |
| Dichlorodifluoromethane | Appropriate amount |

The agent according to the invention is mixed with oleic acid, filled into an aerosol bottle in doses, and injected with dichlorodifluoromethane under pressure.

The invention has been illustrated through description and examples for the purposes of clarity and understanding. It can be understood that the above description and examples are only illustrative rather than restrictive, and various modifications and changes may be made within the scope of the present invention. Accordingly, the scope of the present invention should not be limited to the above description and examples, but should depend on the accompanying claims toghethe with the full scope of equivalents to satisfy these claims.

Patents, patent applications, and scientific literatures cited herein are incorporated herein in their entirety to the same extent as if each were specifically and individually indicated. Any discrepancy between any reference cited herein and the specific teachings of the specification shall be resolved in favor of the latter. Likewise, any discrepancy between the definition of a word or expression as understood in the art and the definition of that word or exression specifically taught in the specification shall also be resolved in favor of the latter.

## Claims

1. Use of folic acid or a pharmaceutically acceptable salt or ester or conjugate thereof in the manufacture of a medicament for inducing B-cell immune tolerance, especially for reducing the production of the antibodies against an immunogenic substance, and/or for the treatment or prevention of a disease or disorder that may be mediated by B-cell immune tolerance.

2. Use according to claim 1, wherein the folic acid or a pharmaceutically acceptable salt or ester or conjugate thereof is selected from the group consisting of free folic acid, a pharmaceutically acceptable salt of folic acid, a pharmaceutically acceptable ester of folic acid, and a pharmaceutically acceptable conjugate of folic acid (e.g., folate-albumin conjugate, folate-polyethylene glycol conjugate, etc.), or any combination thereof.

3. Use according to claim 1 or 2, wherein the B-cell is the one expressing mIgM, especially the one highly expressing mIgM, more especially spleen B-cell or lymph node B-cell.

4. Use according to anyone of claims 1-3, wherein the immunogenic substance is a biomacromolecular active agent, and the medicament is administered in combination with the biomacromolecular active agent, *e.g.,* before and/or during the administration of the biomacromolecular active agent, to reduce the production of Anti-Drug Antibodies against the biomacromolecule active agent;
preferably, the biomacromolecule active agent is selected from the group consisting of: polypeptide drugs, *e.g.,* p53 activating peptide, melittin, scorpion venom peptide, antibacterial peptide, or insulin; or protein drugs, *e.g.,* antibody drugs and especially monoclonal or polyclonal antibody drugs, interferons, growth factors, growth factor inhibitors, enzymes, or albumins, *e.g.,* human serum albumin or ovalbumin, including murine monoclonal antibodies, chimeric monoclonal antibodies, humanized monoclonal antibodies, or fully human monoclonal antibodies, *e.g.,* Tumor Necrosis Factor α (TNFα) mAbs (*e.g.,* adalimumab, etanercept or infliximab), PD1/PD-L1 mAbs (*e.g.,* nivolumab, pembrolizumab, atezolizumab, sintilimab, toripalimab, or camrelizumab); HER2 mAbs (*e.g.,* trastuzumab, pertuzumab, or lapatinib); CD20 mAbs (*e.g.,* rituximab, ibritumomab tiuxetan, or tositumomab); Vascular Endothelial Growth Factor/Vascular Endothelial Growth Factor Receptor (VEGF/VEGFR) mAbs (*e.g.,* bevacizumab, ranibizumab, aflibercept, or ramucirumab); and Epidermal Growth Factor Receptor (EGFR) mAbs (*e.g.,* cetuximab, panitumumab, or necitumumab);
more preferably, the biomacromolecule active agent is selected from the group consisting of adalimumab, infliximab, atezolizumab, sintilimab, toripalimab, trastuzumab, albumins and insulin.

5. Use according to anyone of claims 1-3, wherein the immunogenic substance is an immunogenic drug delivery system, *e.g.,* a microcarrier drug delivery system, preferably selected from the group consisting of liposomes, microspheres, microcapsules, nanoparticles, nanocapsules, lipid nanodiscs or polymer micelles, and wherein the medicament is administered in combination with the drug delivery system, *e.g.,* before and/or during the administration of the drug delivery system, to reduce the production of antibodies against the immunogenic drug delivery system; preferaly, the drug delivery system is loaded with the biomacromolecule active agent as defined in claim 4.

6. Use according to anyone of claims 1-3, wherein the medicament is used for the treatment or prevention of a disease or disorder that may be mediated by B-cell immune tolerance, *e.g.,* hypersensitivity, autoimmune disease or transplant rejection; preferably, the hypersensitivity is selected from the group consisting of anaphylactic shock, respiratory hypersensitivity (e.g., allergic asthma or allergic rhinitis) and gastrointestinal tract hypersensitivity; the autoimmune disease is selected from the group consisting of systemic lupus erythematosus, rheumatoid arthritis, Hashimoto's thyroiditis, toxic diffuse goiter, ankylosing spondylitis, autoimmune encephalomyelitis, neuromyelitis optica spectrum disorders, anticardiolipin syndrome, hemophilia and psoriasis; and the transplant rejection is selected from the group consisting of organ transplant rejection, tissue transplant rejection (*e.g.,* bone marrow transplant rejection) and recurrent miscarriage.

7. Use of folate-modified immunogenic substance in the manufacture of a medicament for inducing B-cell immune tolerance, especially for reducing the production of the antibodies against an immunogenic substance, and/or for the treatment or prevention of a disease or disorder that may be mediated by B-cell immune tolerance.

8. Use according to claim 7, wherein the B-cell is the one expressing mIgM, especially the one highly expressing mIgM, more especially spleen B-cell or lymph node B-cell.

9. Use according to claim 7 or 8, wherein the immunogenic substance is a biomacromolecule active agent;
preferably, the biomacromolecule active agent is selected from the group consisting of: polypeptide drugs, *e.g.,* p53 activating peptide, melittin, scorpion venom peptide, antibacterial peptide, or insulin; or protein drugs, *e.g.,* antibody drugs and especially monoclonal or polyclonal antibody drugs, interferons, growth factors, growth factor inhibitors, enzymes, or albumins, *e.g.,* human serum albumin or ovalbumin, including murine monoclonal antibodies, chimeric monoclonal antibodies, humanized monoclonal antibodies, or fully human monoclonal antibodies, e.g., Tumor Necrosis Factor α (TNFα) mAbs (*e.g.,* adalimumab, etanercept or infliximab), PD1/PD-L1 mAbs (*e.g.,* nivolumab, pembrolizumab, atezolizumab, sintilimab, toripalimab, or camrelizumab); HER2 mAbs (*e.g.,* trastuzumab, pertuzumab, or lapatinib); CD20 mAbs (*e.g.,* rituximab, ibritumomab tiuxetan, or tositumomab); Vascular Endothelial Growth Factor/Vascular Endothelial Growth Factor Receptor (VEGF/VEGFR) mAbs (*e.g.,* bevacizumab, ranibizumab, aflibercept, or ramucirumab); and Epidermal Growth Factor Receptor (EGFR) mAbs (*e.g.,* cetuximab, panitumumab, or necitumumab);
more preferably, the biomacromolecule active agent is selected from the group consisting of adalimumab, infliximab, atezolizumab, sintilimab, toripalimab, trastuzumab, albumins and insulin.

10. Use according to claim 7 or 8, wherein the immunogenic substance is a pathogenic antigenic substance causing an abnormal immune response in the body, preferably a pathogenic antigenic substance causing hypersensitivity, autoimmune disease or transplant rejection, *e.g.,* ovalbumin, proteolipid protein polypeptide, Dby polypeptide, Uty, myelin basic protein, denatured IgG, thyroglobulin, thyrotropin receptor, histone, acetylcholine receptor, major histocompatibility antigens (including HLA-I and HLA-II), minor histocompatibility antigens, blood group antigens, tissue-specific antigens (*e.g.,* vascular endothelial cell antigens, skin SK antigens).

11. Use according to claim 10, wherein the medicament is used for the treatment or prevention of hypersensitivity, autoimmune disease or transplant rejection; preferably, the hypersensitivity is selected from the group consisting of anaphylactic shock, respiratory hypersensitivity (e.g., allergic asthma or allergic rhinitis) and gastrointestinal tract hypersensitivity; the autoimmune disease is selected from the group consisting of systemic lupus erythematosus, rheumatoid arthritis, Hashimoto's thyroiditis, toxic diffuse goiter, ankylosing spondylitis, autoimmune encephalomyelitis, neuromyelitis optica spectrum disorders, anticardiolipin syndrome, hemophilia and psoriasis; and the transplant rejection is selected from the group consisting of organ transplant rejection, tissue transplant rejection (*e.g.,* bone marrow transplant rejection) and recurrent miscarriage.

12. Use of folate-modified antitumor agent or folate-modified probe in the manufacture of a medicament for the targeted diagnosis, treatment or prevention of mIgM-positively-expressed B-cell lymphoma.

13. Use according to claim 12, wherein the antitumor agent in the folate-modified antitumor agent is an antitumor agent useful for treating or preventing mIgM-positively-expressed B-cell lymphoma, preferably anthracyclines, *e.g.,* adriamycin or epirubicin; taxanes, *e.g.,* paclitaxel, docetaxel or cabazitaxel; camptothecins, *e.g.,* camptothecin, hydroxycamptothecin, 9-nitrocamptothecin or irinotecan; vinblastine drugs, *e.g.,* vincristine or vinorelbine; proteasome inhibitors, *e.g.,* bortezomib or carfilzomib; lactone drugs, *e.g.,* parthenolide; cyclophosphamides, etoposide, gemcitabine, cytarabine, 5-fluorouracil, teniposide, mubritinib, epothilone, actinomycin D, mitoxantrone, mitomycin, bleomycin, cisplatin, oxaliplatin, p53 activating peptide, melittin, scorpion venom peptide, triptolide, bevacizumab or trastuzumab; and wherein the probe in the folate-modified probe is a contrast agent, preferably fluorescent substance, *e.g.,* fluorescein, carboxyfluorescein (FAM), fluorescein isothiocyanate (FITC), hexachlorofluorescein (HEX), coumarin 6, near-infrared dyes Cy5, Cy5.5, Cy7, ICG, IR820, DiR or DiD; or radioactive substance, *e.g.,* magnetic resonance contrast agent, *e.g.,* Gd-DTPA or radiocontrast agent, *e.g.,* ⁹⁹mTc-DTPA.

14. A method for inducing B-cell immune tolerance, especially for reducing the production of the antibodies against an immunogenic substance, and/or for the treatment or prevention of a disease or disorder that may be mediated by B-cell immune tolerance, comprising administrating an effective amount of folic acid or a pharmaceutically acceptable salt or ester or conjugate thereof as defined in anyone of claims 1-6 and/or folate-modified immunogenic substance as defined in anyone of claims 7-11 to a subj ect.

15. A method for the targeted diagnosis, treatment or prevention of mIgM-positively-expressed B-cell lymphoma, comprising administering an effective amount of folate-modified antitumor agent or folate-modified probe as defined in claim 12 or 13 to a subject.

16. Folic acid or a pharmaceutically acceptable salt or ester or conjugate thereof as defined in anyone of claims 1-6 and/or folate-modified immunogenic substance as defined in anyone of claims 7-11, especially folate-albumin conjugate, folate-polyethylene glycol conjugate, folate-modified human serumn albumin, folate-modified insulin, folate-modified adalimumab, folate-modified infliximab, folate-modified atezolizumab or folate-modified trastuzumab, for inducing B-cell immune tolerance, especially for reducing the production of the antibodies against an immunogenic substance, and/or for the treatment or prevention of a disease or disorder that may be mediated by B-cell immune tolerance.

17. Folate-modified antitumor agent or folate-modified probe, especially folate-modified cabazitaxel or folate-modified triptolide as defined in claim 12 or 13, for the targeted diagnosis, treatment or prevention of mIgM-positively-expressed B-cell lymphoma.

18. A pharmaceutical composition comprising an effective amount of folic acid or a pharmaceutically acceptable salt or ester or conjugate thereof as defined in anyone of claims 1-6 and/or folate-modified immunogenic substance as defined in anyone of claims 7-11 and/or folate-modified antitumor agent or folate-modified probe as defined in claims 12 or 13, and optionally one or more pharmaceutically acceptable excipients.

19. A combination of folic acid or a pharmaceutically acceptable salt or ester or conjugate thereof with an immunogenic substance, wherein the immunogenic substance is preferably a biomacromolecule active agent or an immunogenic drug delivery system;
wherein, more preferably, the biomacromolecule active agent is selected from the group consisting of: polypeptide drugs, *e.g.,* p53 activating peptide, melittin, scorpion venom peptide, antibacterial peptide, or insulin; or protein drugs, *e.g.,* antibody drugs and especially monoclonal or polyclonal antibody drugs, interferons, growth factors, growth factor inhibitors, enzymes, or albumins, *e.g.,* human serum albumin or ovalbumin, including murine monoclonal antibodies, chimeric monoclonal antibodies, humanized monoclonal antibodies, or fully human monoclonal antibodies, *e.g.,* Tumor Necrosis Factor α (TNFα) mAbs (*e.g.,* adalimumab, etanercept, or infliximab), PD1/PD-L1 mAbs (*e.g.,* nivolumab, pembrolizumab, atezolizumab, sintilimab, toripalimab, or camrelizumab); HER2 mAbs (*e.g.,* trastuzumab, pertuzumab, or lapatinib); CD20 mAbs (*e.g.,* rituximab, ibritumomab tiuxetan, or tositumomab); Vascular Endothelial Growth Factor/Vascular Endothelial Growth Factor Receptor (VEGF/VEGFR) mAbs (*e.g.,* bevacizumab, ranibizumab, aflibercept or ramucirumab); and Epidermal Growth Factor Receptor (EGFR) mAbs (*e.g.,* cetuximab, panitumumab or necitumumab); still more preferably, the biomacromolecule active agent is selected from the group consisting of adalimumab, infliximab, atezolizumab, sintilimab, toripalimab, trastuzumab, albumins and insulin;
or
wherein, more preferably, the immunogenic drug delivery system is microcarrier drug delivery system, e.g., liposome, microsphere, microcapsule, nanoparticle, nanocapsule, lipid nanodisc or polymer micelle.
